# EUROPEAN PATENT SPECIFICATION

(11) **EP 3 855 184 B1**
(45) Date of publication and mention of the grant of the patent: **27.12.2023**
(21) Application number: 20211725.5
(22) Date of filing: 19.03.2013
(51) Int. Cl.: G01N 33/574

(54) **METHODS FOR TREATING AND MONITORING THE STATUS OF CANCER**
VERFAHREN ZUR BEHANDLUNG UND ÜBERWACHUNG EINES KARZINOMSTATUS
PROCÉDÉS DE TRAITEMENT ET DE SURVEILLANCE DE L'ÉTAT D'UN CANCER

(30) Priority: 19.03.2012 US 201261612826 P
(43) Date of publication of application: 28.07.2021
(62) Divisional of application: 18189485.8
(73) Proprietor: Stemline Therapeutics Inc., New York, NY 10022 (US)
(72) Inventor: CIRRITO, Thomas P., Long Island City New York, NY 11109 (US); BERGSTEIN, Ivan, New York,NY 10022 (US); BROOKS, Christopher, Brooklyn New York , NY 11215-5406 (US)
(74) Representative: Graham Watt & Co LLP

(56) References cited:
- US-A1- 2008 175 870
- US-A1- 2011 229 504
- US-A1- 2012 052 080
- BRIAN J MORRISON ET AL: "Breast cancer stem cells: implications for therapy of breast cancer", BREAST CANCER RESEARCH, vol. 10, no. 4, 22 July 2008 (2008-07-22), pages 1-14, XP055218654, GB ISSN: 1465-5411, DOI: 10.1186/bcr2111
- M. DE LA LUZ GARCIA-HERNANDEZ ET AL: "Prostate Stem Cell Antigen Vaccination Induces a Long-term Protective Immune Response against Prostate Cancer in the Absence of Autoimmunity", CANCER RESEARCH, vol. 68, no. 3, 1 February 2008 (2008-02-01), pages 861-869, XP055218655, US ISSN: 0008-5472, DOI: 10.1158/0008-5472.CAN-07-0445
- MADHAV V. DHODAPKAR ET AL: "Vaccines Targeting Cancer Stem Cells", THE CANCER JOURNAL, vol. 17, no. 5, 1 September 2011 (2011-09-01), pages 397-402, XP055218375, ISSN: 1528-9117, DOI: 10.1097/PPO.0b013e318233e730
- HATANO M ET AL: "EPHA2 AS A GLIOMA-ASSOCIATED ANTIGEN: A NOVEL TARGET FOR GLIOMA VACCINES", NEOPLASIA, NEOPLASIA PRESS, ANN ARBOR, MI, US, vol. 7, no. 8, 1 August 2005 (2005-08-01), pages 717-722, XP008076099, ISSN: 1522-8002, DOI: 10.1593/NEO.05277
- TOSHIO FUJISAWA ET AL: "Targeting IL-13R[alpha]2 in human pancreatic ductal adenocarcinoma with combination therapy of IL-13-PE and gemcitabine", INTERNATIONAL JOURNAL OF CANCER, vol. 128, no. 5, 1 March 2011 (2011-03-01) , pages 1221-1231, XP055236401, US ISSN: 0020-7136, DOI: 10.1002/ijc.25437
- VAN NGUYEN ET AL: "IL-13R[alpha]2-Targeted Therapy Escapees: Biologic and Therapeutic Implications", TRANSLATIONAL ONCOLOGY, vol. 4, no. 6, 1 December 2011 (2011-12-01), pages 390-400, XP055236405, United States ISSN: 1936-5233, DOI: 10.1593/tlo.11175
- C. E. BROWN ET AL: "Stem-like Tumor-Initiating Cells Isolated from IL13R 2 Expressing Gliomas Are Targeted and Killed by IL13-Zetakine-Redirected T Cells", CLINICAL CANCER RESEARCH, vol. 18, no. 8, 8 March 2012 (2012-03-08), pages 2199-2209, XP055218517, US ISSN: 1078-0432, DOI: 10.1158/1078-0432.CCR-11-1669

## Description

### 1. INTRODUCTION

The invention is defined by the appended claims. Described herein are methods for treating cancer in a subject comprising administering to the subject a therapeutically effective amount of a peptide derived from the EphA2 protein or the IL-13Rα2 protein and monitoring the amount of cancer stem cells in said subject. Also described are methods for monitoring the efficacy of an EphA2 peptide-based cancer treatment or an IL-13Rα2 peptide-based cancer treatment in a patient with cancer, comprising monitoring the amount of cancer stem cells in said subject prior to, during, and/or following cancer treatment of a patient.

### 2. BACKGROUND

Conventional cancer therapies include surgery, chemotherapy, and radiation therapy. Despite the existence of these therapies, as well as the significant amount of scientific and medical research dedicated annually to uncovering cancer therapeutics, cancer remains one of the leading causes of mortality and morbidity worldwide today. As such, there remains a need for new and effective cancer therapeutics, as well as methods for monitoring the efficacy of existing and new cancer therapeutics. In the prior art Brian J Morrison et al, Breast Cancer Research, vol. 10 no. 4 22 July 2008, pages 1-14 discloses a review on the implications of breast cancer stem cells for therapy of breast cancer. M. De La Luz Garcia-Hernandez et al, Cancer Research, vol. 68, no. 3, 1 February 2008, pages 861-869 discloses prostate stem cell antigen vaccination inducing a long-term protective immune response against prostate cancer in the absence of autoimmunity. Madhav V. Dhodapkar et al, The Cancer Journal, vol. 17, no. 5, 1 September 2011, pages 397-402 discloses vaccines targeting cancer stem cells. Hatano M et al, Neoplasia, vol. 7, no. 8, 1 August 2005, pages 717-722 discloses EphA2 as a Glioma-associated antigen. US 2008/0175870 A1discloses methods for characterizing, isolating and culturing human cancer stem cells. US 2011/0229504 A1 disclsoes peptides, nucleic acids and cells for use in immunotherapeutic methods. Toshio Fujisawa et al, International Journal of Cancer, vol. 128, no. 5, 1 March 2011, pages 1221-1231 discloses targeting IL-13Rα2 in human pancreatic ductal adenocarcinoma within combination therapy of IL-13-PE and gemcitabine. Van Nguyen et al, Translational Oncology, vol. 4, no. 6, 1 December 2011, pages 390-400 discloses IL-13Rα2-targeted therapy escapees; biologic and therapeutic implications. C.E. Brown et al, Clinical Cancer Research, vol. 18, no. 8, 8 March 2012, pages 2199-2209 discloses stem-like tumor initiating cells isolated from IL-13Rα2-expressing gliomas that are targeted and killed by IL-13-zetakine redirected T cells. US 2012/052080 A1 discloses IL-13Rα2 peptide-based brain cancer vaccines.

### 3. SUMMARY

Described herein are methods of treating cancer in a subject, comprising administering to said subject a therapeutically effective amount of a peptide derived from the EphA2 protein and monitoring the amount of cancer stem cells in said subject. The amount of cancer stem cells in said subject that express EphA2 may be measured. The amount of cancer stem cells in said subject that express CD133 may be measured. The amount of cancer stem cells in said subject that express EphA2 and CD133 may be measured.

Methods are described for monitoring the efficacy of an EphA2 peptide-based cancer treatment (i.e., a treatment or therapy that comprises administration of a peptide derived from EphA2) for a patient with cancer, comprising monitoring the amount of cancer stem cells in said subject prior to, during, and/or following the cancer treatment of a patient. The amount of cancer stem cells in said subject that express EphA2 may be measured. The amount of cancer stem cells in said subject that express CD133 may be measured. The amount of cancer stem cells in said subject that express EphA2 and CD133 may be measured.

Also described herein are methods for treating cancer comprising administering a T cell epitope of EphA2 that targets cancer stem cells, wherein said epitope is sufficient to induce an immune response in a patient with cancer.

Also described herein are methods of treating cancer in a subject, comprising administering to said subject a therapeutically effective amount of a compound that targets the EphA2 protein, wherein said compound is capable of killing and/or preventing the differentiation of cancer stem cells that express the EphA2 protein. The compound may be an antibody that specifically binds EphA2.

Described herein are methods of improving the targeting of cancer stem cells with a cancer vaccine comprising determining the binding motif of a Class I or Class II epitope from EphA2, and making substitutions in the amino acid sequence such that the modified peptides are able to induce an immune response that is at least as effective at killing cancer stem cells as the wild type peptide.

Described herein are methods of treating cancer in a subject, comprising administering to said subject a therapeutically effective amount of a peptide derived from the IL-13Rα2 protein and monitoring the amount of cancer stem cells in said subject. The amount of cancer stem cells in said subject that express IL-13Rα2 may be measured. The amount of cancer stem cells in said subject that express CD133 may be measured. The amount of cancer stem cells in said subject that express IL-13Rα2 and CD133 may be measured.

Also described herein are methods for monitoring the efficacy of an IL-13Rα2 peptide-based cancer treatment (i.e., a treatment or therapy that comprises administration of a peptide derived from IL-13Rα2) for a patient with cancer, comprising monitoring the amount of cancer stem cells in said subject prior to, during, and/or following the cancer treatment of a patient. The amount of cancer stem cells in said subject that express IL-13Rα2 may be measured. The amount of cancer stem cells in said subject that express CD133 may be measured. The amount of cancer stem cells in said subject that express IL-13Rα2 and CD133 may be measured.

Described herein are methods for treating cancer comprising administering a T cell epitope of IL-13Rα2 that targets cancer stem cells, wherein said epitope is sufficient to induce an immune response in a patient with cancer.

Also described herein are methods of treating cancer in a subject, comprising administering to said subject a therapeutically effective amount of a compound that targets the IL-13Rα2 protein, wherein said compound is capable of killing and/or preventing the differentiation of cancer stem cells that express the IL-13Rα2 protein. The compound may be an antibody that specifically binds IL-13Rα2.

Described herein are methods of improving the targeting of cancer stem cells with a cancer vaccine comprising determining the binding motif of a Class I or Class II epitope from IL-13Rα2, and making substitutions in the amino acid sequence such that the modified peptides are able to induce an immune response that is at least as effective at killing cancer stem cells as the wild type peptide.

### 3.1 DEFINITIONS

As used herein, the terms "about" or "approximately" when used in conjunction with a number refer to any number within 1, 5 or 10% of the referenced number.

As used herein, the term "agent" refers to any molecule, compound, and/or substance that can be used in or in combination with a method treatment described herein. The term agent includes, without limitation, proteins, immunoglobulins (e.g., multi-specific Igs, single chain Igs, Ig fragments, polyclonal antibodies and their fragments, monoclonal antibodies and their fragments), peptides (e.g., peptide receptors, selectins), binding proteins, biologics, chemospecific agents, chemotoxic agents, anti-angiogenic agents, and small molecule drugs.

As used herein, the term "peptide" refers to a polymer of amino acids linked by amide bonds as is known to those of skill in the art. A peptide can be a polymer of 4, 5, 6, 7, 8, 9, 10, 15, 20, 25, 30, 35, 40, 45, 50, 55, 60, 65, 70, 75, 80, 85, 90,. 95, 100 or more amino acids linked by covalent amide bonds. The peptide may be a polymer of 6 to 8, 8 to 10, 10 to 15, 10 to 20, 10 to 25, 10 to 30, 10 to 40, 10 to 50, or 25 to 25 amino acids linked by covalent amide bonds. The peptide may be a polymer of 50 to 65, 50 to 75, 50 to 85,
50 to 95, 50 to 100, 75 to 100 amino acids linked by covalent amide bonds. As used herein, the term can refer to a single peptide chain linked by covalent amide bonds. The term can also refer to multiple peptide chains associated by non-covalent interactions such as ionic contacts, hydrogen bonds, Van der Waals contacts and hydrophobic contacts. Those of skill in the art will recognize that the term includes peptides that have been modified, for example by post-translational processing such as signal peptide cleavage, disulfide bond formation, glycosylation (*e.g.*, *N*-linked glycosylation), protease cleavage and lipid modification *(e.g.* S-palmitoylation).

As used herein, the terms "purified" and "isolated" when used in the context of a peptide that is obtained from a natural source, *e.g.*, cells, refers to a peptide which is substantially free of contaminating materials from the natural source, *e.g.,* soil particles, minerals, chemicals from the environment, and/or cellular materials from the natural source, such as but not limited to cell debris, cell wall materials, membranes, organelles, the bulk of the nucleic acids, carbohydrates, proteins, and/or lipids present in cells. Thus, a peptide that is isolated includes preparations of a polypeptide having less than about 30%, 20%, 10%, 5%, 2%, or 1% (by dry weight) of cellular materials and/or contaminating materials. As used herein, the terms "purified" and "isolated" when used in the context of a peptide that is chemically synthesized refers to a peptide which is substantially free of chemical precursors or other chemicals which are involved in the syntheses of the polypeptide.

As used herein, the term "nucleic acid" is intended to include DNA molecules (*e.g*., cDNA or genomic DNA) and RNA molecules (*e.g*., mRNA) and analogs of the DNA or RNA generated using nucleotide analogs. The nucleic acid can be single-stranded or double-stranded.

As used herein, the term "therapeutically effective regimen" refers to a regimen for dosing, timing, frequency, and duration of the administration of one or more therapies for the treatment and/or management of cancer or a symptom thereof.

As used herein, the terms "subject" or "patient" are used interchangeably to refer to an animal (*e.g.*, birds, reptiles, and mammals). A subject may be a bird. A subject may be a mammal including a non-primate (*e.g.*, a camel, donkey, zebra, cow, pig, horse, goat, sheep, cat, dog, rat, and mouse) and a primate (*e.g.*, a monkey, chimpanzee, and a humanA subject may be a non-human animal. A subject may be a farm animal or pet. A subject may be a human. A subject may be a human infant. A subject may be a human toddler. A subject may be a human child, a human adult, or an elderly human.

As used herein, the term "brain cancer" refers to a tumor located inside the cranium or in the central spinal canal. Brain cancer refers to both primary tumors (i.e., tumors that originate in the intracranial sphere or the central spinal canal) and secondary tumors (i.e., tumors that invaded the intracranial sphere or the central spinal canal after originating from tumors primarily located in other organs).

As used herein, the terms "therapies" and "therapy" can refer to any protocol(s), method(s), composition(s), formulation(s), and/or agent(s) that can be used in the prevention or treatment of brain cancer or a disease or symptom associated therewith. The terms "therapies" and "therapy" refer to biological therapy, supportive therapy, and/or other therapies useful in treatment or prevention of cancer or a disease or symptom associated therewith known to one of skill in the art.

As used herein, the term "therapeutically effective amount" refers to the amount of a therapy that is sufficient to result in the prevention of the development, recurrence, or onset of cancer and one or more symptoms thereof, to enhance or improve the prophylactic effect(s) of another therapy, reduce the severity, the duration of cancer, ameliorate one or more symptoms of cancer, prevent the advancement of cancer, cause regression of cancer, and/or enhance or improve the therapeutic effect(s) of another therapy. The amount of a therapeutically effective amount may be effective to achieve one, two, three, or more results following the administration of one, two, three or more therapies: (1) a stabilization, reduction or elimination of the cancer stem cell population; (2) a stabilization, reduction or elimination in the cancer cell population; (3) a stabilization or reduction in the growth of a tumor or neoplasm; (4) an impairment in the formation of a tumor; (5) eradication, removal, or control of primary, regional and/or metastatic cancer; (6) a reduction in mortality; (7) an increase in disease-free, relapse-free, progression-free, and/or overall survival, duration, or rate; (8) an increase in the response rate, the durability of response, or number of patients who respond or are in remission; (9) a decrease in hospitalization rate, (10) a decrease in hospitalization lengths, (11) the size of the tumor is maintained and does not increase or increases by less than 10%, preferably less than 5%, preferably less than 4%, preferably less than 2%, (12) an increase in the number of patients in remission, (13) an increase in the length or duration of remission, (14) a decrease in the
recurrence rate of cancer, (15) an increase in the time to recurrence of cancer, and (16) an amelioration of cancer-related symptoms and/or quality of life.

As used herein, the term "in combination" in the context of the administration of a therapy to a subject refers to the use of more than one therapy (*e.g.*, prophylactic and/or therapeutic). The use of the term "in combination" does not restrict the order in which the therapies (*e.g*., a first and second therapy) are administered to a subject. A therapy can be administered prior to (*e.g.*, 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.*, 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy to a subject which had, has, or is susceptible to brain cancer. The therapies are administered to a subject in a sequence and within a time interval such that the therapies can act together. The therapies may be administered to a subject in a sequence and within a time interval such that they provide an increased benefit than if they were administered otherwise. Any additional therapy can be administered in any order with the other additional therapy.

As used herein, the terms "manage," "managing," and "management" in the context of the administration of a therapy to a subject refer to the beneficial effects that a subject derives from a therapy (*e.g*., a prophylactic or therapeutic vaccine) or a combination of therapies, while not resulting in a cure of cancer. A subject may be administered one or more therapies (*e.g*., one or more prophylactic or therapeutic vaccines) to "manage" cancer so as to prevent the progression or worsening of the condition.

As used herein, the terms "prevent," "preventing" and "prevention" in the context of the administration of a therapy to a subject refer to the prevention or inhibition of the recurrence, onset, and/or development of brain cancer or a symptom thereof in a subject resulting from the administration of a therapy (*e.g*., a prophylactic or therapeutic agent), or a combination of therapies (*e.g*., a combination of prophylactic or therapeutic agents).

As used herein, the term "concurrently" means sufficiently close in time to produce a combined effect (that is, concurrently may be simultaneously, or it may be two or more events occurring within a time period before or after each other). When administered with other agents, the EphaA2 and/or IL-13Rα2 peptides provided herein may be administered concurrently with the other active agent. In some embodiments the EphaA2 and/or IL-13Rα2 peptides provided herein and one or more other agents (e.g., a helper T cell epitope, an adjuvant, and/or an immune response modifier) are administered to a subject concurrently, wherein administration of the EphaA2 and/or IL-13Rα2 peptide and one or more other agents are in the same composition. In other embodiments an EphaA2 and/or IL-13Rα2 peptide and one or more other agents (e.g., a helper T cell epitope, an adjuvant, and/or an immune response modifier) are administered to a subject concurrently, wherein administration of the EphaA2 and/or IL-13Rα2 peptide and one or more other agents are not in the same composition. In certain embodiments, an EphaA2 peptide and/or IL-13Rα2 provided herein and one or more other agents (e.g., a helper T cell epitope, an adjuvant, and/or an immune response modifier) are administered to a subject concurrently, wherein the concurrent administration is separated by at least 1 hour, 2 hours, 3 hours, 4 hours, 5 hours, 10 hours, 12 hours, 1 day, 2 days, 3 days, 4 days, 5 days, 6 days, 1 week, or 2 weeks.

As used herein, the term "EphA2 peptide" refers to a peptide derived from the EphA2 protein. In a specific embodiment the EphA2 protein from which an EphA2 peptide is derived is the human EphA2 protein. In another specific embodiment, an EphA2 peptide comprises or consists of the following amino acid sequence: TLADFDPRV (SEQ ID NO:1). In some embodiments, an EphA2 peptide comprises one, two, three, or more amino acid mutations (e.g., additions, substitutions, or deletions) relative to the EphA2 peptide as it exists in the native (e.g., wild-type) form of the EphA2 protein.

As used herein, the term "IL-13Rα2 peptide" refers to a peptide derived from the IL-13Rα2 protein. In a specific embodiment the IL-13Rα2 protein from which an IL-13Rα2 peptide is derived is the human IL-13Rα2 protein. In another specific embodiment, an IL-13Rα2 peptide comprises or consists of the following amino acid sequence: WLPFGFILI (SEQ ID NO:2). In another specific embodiment, an IL-13Rα2 peptide comprises or consists of the following amino acid sequence: WLPFGFILV (SEQ ID NO:3). In another specific embodiment, an IL-13Rα2 peptide comprises or consists of the following amino acid sequence: ALPFGFII,V (SEQ ID NO:4). In another specific embodiment, an IL-13Rα2 peptide comprises or consists of the following amino acid sequence: ELPFGFILV (SEQ ID NO:5). In some embodiments, an IL-13Rα2 peptide comprises one, two, three, or more amino acid mutations (e.g., additions, substitutions, or deletions) relative to the IL-13Rα2 peptide as it exists in the native (e.g., wild-type) form of the IL-13Rα2 protein.

As used herein and unless otherwise specified, the term "antibody refers to a molecule with an antigen binding site that immunospecifically binds an antigen. Antibodies include, but are not limited to, monoclonal antibodies, polyclonal antibodies, recombinantly produced antibodies, multispecific antibodies (including bi-specific antibodies), human antibodies, humanized antibodies, chimeric antibodies, synthetic antibodies, tetrameric antibodies comprising two heavy chain and two light chain molecule, an antibody light chain monomer, an antibody heavy chain monomer, an antibody light chain dimer, an antibody heavy chain dimer, an antibody light chain- antibody heavy chain pair, intrabodies, heteroconjugate antibodies, single domain antibodies, monovalent antibodies, single-chain Fvs (scFv) (*e.g*., including monospecific, bispecific, *etc.*), camelized antibodies, Fab fragments, F(ab') fragments, disulfide-linked Fvs (sdFv), anti-idiotypic (anti-Id) antibodies (including, *e.g.*, anti-anti-Id antibodies), and epitope-binding fragments of any of the above. Antibodies can be of any type *(e.g.,* IgG, IgE, IgM, IgD, IgA or IgY), any class, *(e.g.,* IgG1, IgG2, IgG3, IgG4, IgA1 or IgA2), or any subclass (*e.g.*, IgG2a or IgG2b) of immunoglobulin molecule. In certain embodiments, antibodies described herein are IgG antibodies, or a class (*e.g.*, human IgG1 or IgG4) or subclass thereof.

### 4. BRIEF DESCRIPTION OF THE DRAWINGS

**Fig. 1** demonstrate that the bulk of the cells of the A-172 cancer cell line express EphA2 and IL-13Rα2 at high levels, but only a fraction of these cells express CD133.
**Fig. 2** depict joint staining of CD133 and EphA2 cells of the A-172 cancer cell line, and demonstrate that CD133+ cells of the cell line also express EphA2.

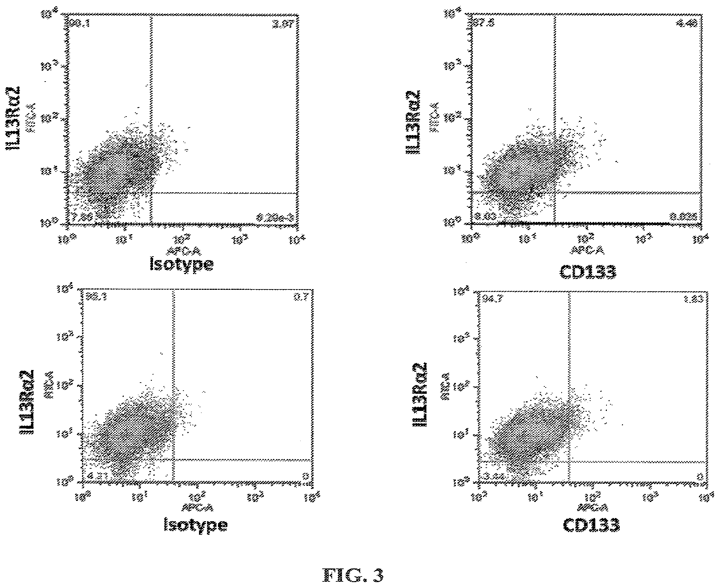

**Fig. 3** depict joint staining of CD133 and IL-13Rα2 cells of the A-172 cancer cell line, and demonstrate that CD133+ cells of the cell line also express IL-13Rα2.
**Fig. 4** shows that CD133+ cells of the A-172 cancer cell line also express EphA2.
**Fig. 5** shows that CD133+ cells of the A-172 cancer cell line also express IL-13Rα2.
**Fig. 6** demonstrate that the bulk of the cells of the A-172 cancer cell line express EphA2 and IL-13Rα2 at high levels, but only a fraction of these cells express CD133.
**Fig. 7** demonstrate that only a fraction of the cells of the A-172 cancer cell line express CD133.
**Fig. 8** demonstrate that CD133+ cells of the A-172 cancer cell line also express EphA2 and IL-13Rα2.

### 5. DETAILED DESCRIPTION

Described herein are methods of treating cancer in a subject, comprising administering to said subject a therapeutically effective amount of a peptide derived from the EphA2 protein and monitoring the amount of cancer stem cells in said subject. The amount of cancer stem cells in said subject that express EphA2 may be measured. The amount of cancer stem cells in said subject that express CD133 may be measured. In another specific embodiment, the amount of cancer stem cells in said subject that express EphA2 and CD133 may be measured.

Also described herein are methods for monitoring the efficacy of an EphA2 peptide-based cancer treatment (i.e., a treatment or therapy that comprises administration of a peptide derived from EphA2) for a patient with cancer, comprising monitoring the amount of cancer stem cells in said subject prior to, during, and/or following the cancer treatment of a patient. The amount of cancer stem cells in said subject that express EphA2 may be measured. The amount of cancer stem cells in said subject that express CD133 may be measured. The amount of cancer stem cells in said subject that express EphA2 and CD133 may be measured.

Described herein are methods for treating cancer comprising administering a T cell epitope of EphA2 that targets cancer stem cells, wherein said epitope is sufficient to induce an immune response in a patient with cancer.

Also described herein are methods of treating cancer in a subject, comprising administering to said subject a therapeutically effective amount of a compound that targets the EphA2 protein, wherein said compound is capable of killing and/or preventing the differentiation of cancer stem cells that express the EphA2 protein. The compound may be an antibody that specifically binds EphA2.

Also described herein are methods of improving the targeting of cancer stem cells with a cancer vaccine comprising determining the binding motif of a Class I or Class II epitope from EphA2, and making substitutions in the amino acid sequence such that the modified peptides are able to induce an immune response that is at least as effective at killing cancer stem cells as the wild type peptide.

Provided herein are methods of treating cancer in a subject, comprising administering to said subject a therapeutically effective amount of a peptide derived from the IL-13Rα2 protein and monitoring the amount of cancer stem cells in said subject. The amount of cancer stem cells in said subject that express IL-13Rα2 may be measured. The amount of cancer stem cells in said subject that express CD133 may be measured. The amount of cancer stem cells in said subject that express IL-13Rα2 and CD133 may be measured.

Provided herein are methods for monitoring the efficacy of an IL-13Rα2 peptide-based cancer treatment (i.e., a treatment or therapy that comprises administration of a peptide derived from IL-13Rα2) for a patient with cancer, comprising monitoring the amount of cancer stem cells in said subject prior to, during, and/or following the cancer treatment of a patient. The amount of cancer stem cells in said subject that express IL-13Rα2 may be measured. The amount of cancer stem cells in said subject that express CD133 may be measured. The amount of cancer stem cells in said subject that express IL-13Rα2 and CD133 may be measured.

Provided herein are methods for treating cancer comprising administering a T cell epitope of IL-13Rα2 that targets cancer stem cells, wherein said epitope is sufficient to induce an immune response in a patient with cancer.

Provided herein are methods of treating cancer in a subject, comprising administering to said subject a therapeutically effective amount of a compound that targets the IL-13Rα2 protein, wherein said compound is capable of killing and/or preventing the differentiation of cancer stem cells that express the IL-13Rα2 protein. The compound may be an antibody that specifically binds IL-13Rα2.

Provided herein are methods of improving the targeting of cancer stem cells with a cancer vaccine comprising determining the binding motif of a Class I or Class II epitope from IL-13Rα2, and making substitutions in the amino acid sequence such that the modified peptides are able to induce an immune response that is at least as effective at killing cancer stem cells as the wild type peptide.

### 5.1 METHODS OF MONITORING CANCER STEM CELLS

As part of the prophylactically effective and/or therapeutically effective regimens described herein, cancer stem cells can be monitored to assess the efficacy of an EphA2 peptide based cancer therapy or an IL-13Rα2 peptide based cancer therapy as well as to determine prognosis of a subject with cancer or the efficacy of a therapeutically or prophylactically effective regimen. In the prophylactically effective and/or therapeutically effective therapies or regimens described herein, the therapies or regimens result in a stabilization or reduction of cancer stem cells in the patient. The subject undergoing the regimen is monitored to assess whether the regimen has resulted in a stabilization or reduction in the cancer stem cells in the subject. In specific embodiments, the methods of monitoring measure an EphA2-, IL-13Rα2-, and/or CD133-expressing cancer stem cells in the subjects to whom an an EphA2 peptide based cancer therapy or an IL-13Rα2 peptide based cancer therapy is administered.

Without being limited by any particular theory or mechanism of action, cancer stem cells, e.g., EphA2-expressing cancer stem cells and/or IL-13Rα2-expressing cancer stem cells, comprise a unique subpopulation (e.g., 0.1-10% or so) of a tumor that, in contrast to the remaining 90% or so of the tumor (*i.e.*, the tumor bulk), are relatively more tumorigenic and relatively more slow-growing or quiescent. Given that conventional therapies and regimens have, in large part, been designed to attack rapidly proliferating cells (*i.e.*, those cancer cells that comprise the tumor bulk), slower growing cancer stem cells may be relatively more resistant than faster growing tumor bulk to conventional therapies and regimens. This would explain another reason for the failure of standard oncology treatment regimens to ensure long-term benefit in most patients with advanced stage cancers. An EphA2-expressing cancer stem cell or IL-13Rα2-expressing cancer stem cell is the founder cell of a tumor (*i.e.*, it is the progenitor of cancer cells). An EphA2-expressing cancer stem cell or IL-13Rα2-expressing cancer stem cell has one, two, three, or more or all of the following characteristics or properties: (i) can harbor the ability to initiate a tumor and/or to perpetuate tumor growth, (ii) can be generally relatively less mutated than the bulk of a tumor (*e.g.* due to slower growth and thus fewer DNA replication-dependent errors, improved DNA repair, and/or epigenetic/non-mutagenic changes contributing to their malignancy), (iii) can have many features of a normal stem cell(s) (*e.g.*, similar cell surface antigen and/or intracellular expression profile, self-renewal programs, multi-drug resistance, an immature phenotype, etc., characteristic of normal stem cells) and may be derived from a normal stem cell(s), (iv) can be potentially responsive to its microenvironment (*e.g.*, the cancer stem cells may be capable of being induced to differentiate and/or divide asymmetrically), (v) can be the source of metastases, (vi) can be slow-growing or quiescent, (vii) can be symmetrically-dividing, (viii) can be tumorigenic (*e.g.* as determined by NOD/SCID implantation experiments), (ix) can be relatively resistant to traditional therapies (*i.e.* chemoresistant), and (x) can comprise a subpopulation of a tumor (*e.g.* relative to the tumor bulk).

The amount of cancer stem cells in a sample from a subject may be determined/assessed using a technique described herein or well-known to one of skill in the art. Such samples include, but are not limited to, biological samples and samples derived from a biological sample. In addition to the biological sample itself or in addition to material derived from the biological sample such as cells, the sample used in the methods of this invention comprises added water, salts, glycerin, glucose, an antimicrobial agent, paraffin, a chemical stabilizing agent, heparin, an anticoagulant, or a buffering agent. The biological sample may be blood, serum, urine, bone marrow or interstitial fluid. The sample may be a tissue sample. The tissue sample may be breast, brain, skin, colon, lung, liver, ovarian, pancreatic, prostate, renal, bone or skin tissue. The tissue sample may be a biopsy of normal or tumor tissue. The amount of biological sample taken from the subject will vary according to the type of biological sample and the method of detection to be employed. The biological sample may be blood, serum, urine, or bone marrow and the amount of blood, serum, urine, or bone marrow taken from the subject is 0.1 ml, 0.5 ml, 1 ml, 5 ml, 8 ml, 10 ml or more. The biological sample may be a tissue and the amount of tissue taken from the subject is less than 10 milligrams, less than 25 milligrams, less than 50 milligrams, less than 1 gram, less than 5 grams, less than 10 grams, less than 50 grams, or less than 100 grams.

In accordance with the methods described herein, a sample derived from a biological sample is one in which the biological sample has been subjected to one or more pretreatment steps prior to the detection and/or measurement of the cancer stem cell population in the sample. A biological fluid may be pretreated by centrifugation, filtration, precipitation, dialysis, or chromatography, or by a combination of such pretreatment steps. A tissue sample may be pretreated by freezing, chemical fixation, paraffin embedding, dehydration, permeablization, or homogenization followed by centrifugation, filtration, precipitation, dialysis, or chromatography, or by a combination of such pretreatment steps. The sample may be pretreated by removing cells other than stem cells or cancer stem cells from the sample, or removing debris from the sample prior to the determination of the amount of cancer stem cells in the sample according to the methods of the invention.

The samples for use in the methods described herein may be taken from any animal subject, preferably mammal, most preferably a human. The subject from which a sample is obtained and utilized in accordance with the methods described includes, without limitation, an asymptomatic subject, a subject manifesting or exhibiting 1, 2, 3, 4 or more symptoms of cancer, a subject clinically diagnosed as having cancer, a subject predisposed to cancer, a subject suspected of having cancer, a subject undergoing therapy for cancer, a subject that has been medically determined to be free of cancer (e.g., following therapy for the cancer), a subject that is managing cancer, or a subject that has not been diagnosed with cancer. The term "has no detectable cancer," as used herein, refers to a subject or subjects in which there is no detectable cancer by conventional methods, *e.g*., MRI. In other embodiments, the term refers to a subject or subjects free from any disorder.

The amount of cancer stem cells in a subject or a sample from a subject is/are assessed prior to therapy or regimen (*e.g.* at baseline) or at least 1, 2, 4, 6, 7, 8, 10, 12, 14, 15, 16, 18, 20, 30, 60, 90 days, 6 months, 9 months, 12 months, or > 12 months after the subject begins receiving the therapy or regimen. The amount of cancer stem cells may be assessed after a certain number of doses (*e.g.*, after 2, 5, 10, 20, 30 or more doses of a therapy). In other embodiments, the amount of cancer stem cells is assessed after 1 week, 2 weeks, 1 month, 2 months, 1 year, 2 years, 3 years, 4 years or more after receiving one or more therapies.

A positive or negative control sample is a sample that is obtained or derived from a corresponding tissue or biological fluid or tumor as the sample to be analyzed in accordance with the methods described. This sample may come from the same patient or different persons and at the same or different time points.

For clarity of disclosure, and not by way of limitation, the following pertains to analysis of a blood sample from a patient. However, as one skilled in the art will appreciate, the assays and techniques described herein can be applied to other types of patient samples, including a body fluid (*e.g.* blood, bone marrow, plasma, urine, bile, ascitic fluid), a tissue sample suspected of containing material derived from a cancer (*e.g.* a biopsy) or homogenate thereof. The amount of sample to be collected will vary with the particular type of sample and method of determining the amount of cancer stem cells used and will be an amount sufficient to detect the cancer stem cells in the sample.

A sample of blood may be obtained from a patient having different developmental or disease stages. Blood may be drawn from a subject from any part of the body (*e.g.*, a finger, a hand, a wrist, an arm, a leg, a foot, an ankle, a stomach, and a neck) using techniques known to one of skill in the art, in particular methods of phlebotomy known in the art. Venous blood is obtained from a subject and utilized in accordance with the methods described. Arterial blood may be obtained and utilized in accordance with the methods described. The composition of venous blood varies according to the metabolic needs of the area of the body it is servicing. In contrast, the composition of arterial blood is consistent throughout the body. For routine blood tests, venous blood is generally used.

The amount of blood collected will vary depending upon the site of collection, the amount required for a method described, and the comfort of the subject. Any amount of blood may be collected that is sufficient to detect the amount of cancer stem cells. For example, 1cc or more of blood is collected from a subject.

The amount of cancer stem cells in a sample can be expressed as the percentage of, e.g., overall cells, overall cancer cells or overall stem cells in the sample, or quantitated relative to area (*e.g.* cells per high power field), or volume (*e.g.* cells per ml), or architecture (*e.g.* cells per bone spicule in a bone marrow specimen).

The sample may be a blood sample, bone marrow sample, or a tissue/tumor biopsy sample, wherein the amount of cancer stem cells per unit of volume (*e.g.*, 1 mL) or other measured unit (*e.g.*, per unit field in the case of a histological analysis) is quantitated. The cancer stem cell population may be determined as a portion (*e.g.*, a percentage) of the cancerous cells present in the blood or bone marrow or tissue/tumor biopsy sample or as a subset of the cancerous cells present in the blood or bone marrow or tissue/tumor biopsy sample. The cancer stem cell population, can be determined as a portion (*e.g*., percentage) of the total cells. The cancer stem cell population may be determined as a portion (*e.g.*, a percentage) of the total stem cells present in the blood sample.

The sample from the patient may be a tissue sample (*e.g.*, a biopsy from a subject with or suspected of having cancerous tissue), where the amount of cancer stem cells can be measured, for example, by immunohistochemistry or flow cytometry, or on the basis of the amount of cancer stem cells per unit area, volume, or weight of the tissue. The cancer stem cell population (the amount of cancer stem cells) may be determined as a portion (*e.g.*, a percentage) of the cancerous cells present in the tissue sample or as a subset of the cancerous cells present in the tissue sample. The cancer stem cell population may be determined as a portion (*e.g.*, a percentage) of the overall cells or stem cell cells in the tissue sample.

The amount of cancer stem cells in a test sample can be compared with the amount of cancer stem cells in reference sample(s) to assess the efficacy of the regimen. The reference sample may be a sample obtained from the subject undergoing therapy at an earlier time point (*e.g.*, prior to receiving the regimen as a baseline reference sample, or at an earlier time point while receiving the therapy). In this case, the therapy desirably results in a decrease in the amount of cancer stem cells in the test sample as compared with the reference sample. The reference sample may be obtained from a healthy subject who has no detectable cancer, or from a patient that is in remission for the same type of cancer. In this case, the therapy desirably results in the test sample having an equal amount of cancer stem cells, or less than the amount of cancer stem cells than are detected in the reference sample.

In other embodiments, the cancer stem cell population in a test sample can be compared with a predetermined reference range and/or a previously detected amount of cancer stem cells determined for the subject to gauge the subject's response to the regimens described herein. In a specific embodiment, a stabilization or reduction in the amount of cancer stem cells relative to a predetermined reference range and/or earlier (previously detected) cancer stem cell amount determined for the subject indicates an improvement in the subject's prognosis or a positive response to the regimen, whereas an increase relative to the predetermined reference range and/or earlier cancer stem cell amount indicates the same or worse prognosis, and/or a failure to respond to the regimen. The cancer stem cell amount can be used in conjunction with other measures to assess the prognosis of the subject and/or the efficacy of the regimen. In a specific embodiment, the predetermined reference range is based on the amount of cancer stem cells obtained from a patient or population(s) of patients suffering from the same type of cancer as the patient undergoing the therapy.

Generally, since stem cell antigens, e.g., EphA2, CD133, and IL-13Rα2, can be present on both cancer stem cells and normal stem cells, a sample from the cancer-afflicted patient will have a higher stem cell count than a sample from a healthy subject who has no detectable cancer, due to the presence of the cancer stem cells. The therapy will desirably result in a cancer stem cell count for the test sample (*e.g.*, the sample from the patient undergoing therapy) that decreases and becomes increasingly closer to the stem cell count in a reference sample that is sample from a healthy subject who has no detectable cancer.

If the reduction in amount of cancer stem cells is determined to be inadequate upon comparing the amount of cancer stem cells in the sample from the subject undergoing the regimen with the reference sample, then the medical practitioner has a number of possible options to adjust the regimen. For instance, the medical practitioner can then increase either the dosage or intensity of the therapy administered, the frequency of the administration, the duration of administration, combine the therapy with another therapy(ies), change the management altogether including halting therapy, or any combination thereof.

The dosage, frequency and/or duration of administration of a therapy may be modified as a result of the change in the amount of cancer stem cells detected in or from the treated patient. For example, if a subject receiving therapy for leukemia has a cancer stem cell measurement of 2.5% of his tumor prior to therapy and 5% after 6 weeks of therapy, then the therapy or regimen may be altered or stopped because the increase in the percentage of cancer stem cells indicates that the therapy or regimen is not optimal. Alternatively, if another subject has a cancer stem cell measurement of 2.5% of his tumor prior to therapy and 1% after 6 weeks of therapy, then the therapy or regimen may be continued because the decrease in the percentage of cancer stem cells indicates that the therapy or regimen is effective.

The amount of cancer stem cells can be monitored/assessed using standard techniques known to one of skill in the art. Cancer stem cells can be monitored by, *e.g.*, obtaining a sample, such as a tissue/tumor sample, blood sample or a bone marrow sample, from a subject and detecting cancer stem cells in the sample. The amount of cancer stem cells in a sample (which may be expressed as percentages of, *e.g.*, overall cells or overall cancer cells) can be assessed by detecting the expression cancer stem cell antigens (e.g., EphA2) on cancer stem cells. Techniques known to those skilled in the art can be used for measuring these activities. Antigen expression can be assayed, for example, by immunoassays including, but not limited to, western blots, immunohistochemistry, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, immunofluorescence, protein A immunoassays, flow cytometry, and FACS analysis. In such circumstances, the amount of cancer stem cells in a test sample from a subject may be determined by comparing the results to the amount of cancer stem cells in a reference sample (*e.g.*, a sample from a subject who has no detectable cancer) or to a predetermined reference range, or to the patient him/herself at an earlier time point (*e.g.* prior to, or during therapy).

In a specific embodiment, the cancer stem cell population in a sample from a patient is determined by flow cytometry. This method exploits the differential expression of certain surface markers on cells. Labeled antibodies (*e.g.*, fluorescent antibodies) specific to cancer stem cells antigens (e.g., EphA2) can be used to react with the cells in the sample, and the cells are subsequently sorted by FACS methods. In some embodiments, a combination of cell surface markers are utilized in order to determine the amount of cancer stem cells in the sample. For example, both positive and negative cell sorting may be used to assess the amount of cancer stem cells in the sample. In a specific embodiment the cancer stem cell population in a sample, *e.g.,* a tissue sample, such as a solid tumor biopsy, is determined using immunohistochemistry techniques. This method exploits the differential expression of certain surface markers on cells. Labeled antibodies (e.g., fluorescent antibodies) specific to cancer stem cells antigens (e.g., EphA2) can be used to react with the cells in the sample, and the tissue is subsequently stained. In some embodiments, a combination of certain cell surface markers are utilized in order to determine the amount of cancer stem cells in the sample.

In other embodiments, sphere formation can be used to determine the amount of cancer stem cells in a sample (*See* Singh et al., "Identification of a Cancer Stem Cell from Human Brain Tumors," Cancer Res 63: 5821-5828 (2003).

In other embodiments, a sample (*e.g.*, a tumor or normal tissue sample, blood sample or bone marrow sample) obtained from the patient is analyzed in *in vivo* systems to determine the cancer stem cell population or amount of cancer stem cells. In certain embodiments, for example, *in vivo* engraftment is used to quantitate the amount of cancer stem cells in a sample. *In vivo* engraftment involves implantation of a human specimen with the readout being the formation of tumors in an animal such as in immunocompromised or immunodeficient mice (such as NOD/SCID mice). Typically, the patient sample is cultured or manipulated *in vitro* and then injected into the mice. In these assays, mice can be injected with a decreasing amount of cells from patient samples, and the frequency of tumor formation can be plotted vs. the amount of cells injected to determine the amount of cancer stem cells in the sample. Alternatively, the rate of growth of the resulting tumor can be measured, with larger or more rapidly advancing tumors indicating a higher cancer stem cells amount in the patient sample. In this way, an *in vivo* engraftment model/assay could be used to measure cancer stem cells amount pre- and post-therapy to assess the change in cancer stem cell amount arising from a given therapy or regimen.

In certain *in vivo* techniques, an imaging agent or diagnostic agent is used which binds to biological molecules on cancer cells or cancer stem cells, *e.g.,* binds to EphA2 on cancer stem cells. For instance, a fluorescent tag, radionuclide, heavy metal, or photon-emitter is attached to an antibody (including an antibody fragment) that binds to EphA2. The medical practitioner can infuse the labeled antibody into the patient either prior to, during, or following treatment, and then the practitioner can place the patient into a total body scanner/developer which can detect the attached label (*e.g.*, fluorescent tag, radionuclide, heavy metal, photon-emitter). The scanner/developer (*e.g.*, CT, MRI, or other scanner, e.g. detector of fluorescent label, that can detect the label) records the presence, amount/quantity, and bodily location of the bound antibody. In this manner, the mapping and quantitation of tag (*e.g.* fluorescence, radioactivity, etc.) in patterns (*i.e.*, different from patterns of normal stem cells within a tissue) within a tissue or tissues indicates the treatment efficacy within the patient's body when compared to a reference control such as the same patient at an earlier time point or a patient or healthy individual who has no detectable cancer. For example, a large signal (relative to a reference range or a prior treatment date, or prior to treatment) at a particular location indicates the presence of cancer stem cells. If this signal is increased relative to a prior date it suggests a worsening of the disease and failure of therapy or regimen. Alternatively, a signal decrease indicates that the therapy or regimen has been effective.

The amount of cancer stem cells may be detected in vivo in a subject according to a method comprising the steps of: (a) administering to the subject an effective amount of a labeled binding agent that specifically binds to an antgen of cancer stem cells (e.g., EphA2 or CD133), and (b) detecting the labeled agent in the subject following a time interval sufficient to allow the labeled agent to concentrate at sites in the subject where the cancer stem cell surface marker is expressed. In accordance with this, the binding agent is administered to the subject according to any suitable method in the art, for example, parenterally (such as intravenously), or intraperitoneally. In accordance with this embodiment, the effective amount of the agent is the amount which permits the detection of the agent in the subject. This amount will vary according to the particular subject, the label used, and the detection method employed. For example, it is understood in the art that the size of the subject and the imaging system used will determine the amount of labeled agent needed to detect the agent in a subject using an imaging means. In the case of a radiolabeled agent for a human subject, the amount of labeled agent administered is measured in terms of radioactivity, for example from about 5 to 20 millicuries of 99Tc. The time interval following the administration of the labeled agent which is sufficient to allow the labeled agent to concentrate at sites in the subject where the cancer stem cell surface marker is expressed will vary depending on several factors, for example, the type of label used, the mode of administration, and the part of the subject's body that is imaged. The time interval that is sufficient may be 6 to 48 hours, 6 to 24 hours, or 6 to 12 hours. Alternatively the time interval is 5 to 20 days or 5 to 10 days. The presence of the labeled cancer stem cell surface marker-binding agent can be detected in the subject using imaging means known in the art. In general, the imaging means employed depend upon the type of label used. Skilled artisans will be able to determine the appropriate means for detecting a particular label. Methods and devices that may be used include, but are not limited to, computed tomography (CT), whole body scan such as position emission tomography (PET), magnetic resonance imaging (MRI), an imager which can detect and localize fluorescent label, and sonography. In a specific embodiment, the cancer binding agent is labeled with a radioisotope and is detected in the patient using a radiation responsive surgical instrument (Thurston et al., U.S. Patent No. 5,441,050). The binding agent is labeled with a fluorescent compound and is detected in the patient using a fluorescence responsive scanning instrument. In another embodiment, the binding agent is labeled with a positron emitting metal and is detected in the patient using positron emission-tomography. In yet another embodiment, the binding agent is labeled with a paramagnetic label and is detected in a patient using magnetic resonance imaging (MRI).

Any *in vitro* or *in vivo* (*ex vivo*) assays known to those skilled in the art that can detect and/or quantify cancer stem cells can be used to monitor cancer stem cells in or from a subject in order to evaluate the prophylactic and/or therapeutic utility of a cancer therapy or regimen disclosed herein for cancer or one or more symptoms thereof; or these assays can be used to assess the prognosis of a patient. The results of these assays then may be used to possibly maintain or alter the cancer therapy or regimen.

The amount of cancer stem cells in a specimen can be compared to a predetermined reference range and/or an earlier amount of cancer stem cells previously determined for the subject (either prior to, or during therapy) in order to gauge the subject's response to the treatment regimens described herein. In a specific embodiment, a stabilization or reduction in the amount of cancer stem cells relative to a predetermined reference range and/or earlier cancer stem cell amount previously determined for the subject (either prior to, or during therapy) indicates that the therapy or regimen was effective and thus possibly an improvement in the subject's prognosis, whereas an increase relative to the predetermined reference range and/or cancer stem cell amount detected at an earlier time point indicates that the therapy or regimen was ineffective and thus possibly the same or a worsening in the subject's prognosis. The cancer stem cell amount can be used with other standard measures of cancer to assess the prognosis of the subject and/or efficacy of the therapy or regimen: such as response rate, durability of response, relapse-free survival, disease-free survival, progression-free survival, and overall survival. In certain embodiments, the dosage, frequency and/or duration of administration of a therapy is modified as a result of the determination of the amount or change in the amount of cancer stem cells at various time points which may include prior to, during, and/or following therapy.

Also provided herein are methods for determining that a cancer therapy or regimen is effective at targeting and/or impairing cancer stem cells by virtue of monitoring cancer stem cells over time and detecting a stabilization or decrease in the amount of cancer stem cells during and/or following the course of the cancer therapy or regimen.

A therapy or regimen may be described or marketed as an anti- cancer stem cell therapy or regimen based on the determination that a therapy or regimen is effective at targeting and/or impairing cancer stem cells by virtue of having monitored or detected a stabilization or decrease in the amount of cancer stem cells during therapy.

Also provided herein are methods to treat cancer involving i) determining that an EphA2-based cancer therapy is effective by virtue of its ability to decrease cancer stem cells as determined by the monitoring of cancer stem cells, and ii) administering the therapy to a human(s) with cancer. Also provided herein are methods to methods to treat cancer involving i) administering to a human with cancer an EphA2-based cancer therapy, ii) determining the amount of cancer stem cells prior to, during, and/or following therapy through the monitoring of cancer stem cells, and iii) continuing, altering, or halting therapy based on such monitoring. Also provided herein are methods for assaying/screening of an EphA2-based therapy(s) for anti-cancer stem cell activity involving i) administration of the therapy to a human with cancer, ii) monitoring cancer stem cells in or from the human prior to, during, and/or following therapy, and iii) determining whether the therapy resulted in a decrease in the amount of cancer stem cells.

Also provided herein are methods to treat cancer involving i) determining that an IL-13Rα2-based cancer therapy is effective by virtue of its ability to decrease cancer stem cells as determined by the monitoring of cancer stem cells, and ii) administering the therapy to a human(s) with cancer. Also provided herein are methods to methods to treat cancer involving i) administering to a human with cancer an IL-13Rα2-based cancer therapy, ii) determining the amount of cancer stem cells prior to, during, and/or following therapy through the monitoring of cancer stem cells, and iii) continuing, altering, or halting therapy based on such monitoring. Also provided herein are methods for assaying/screening of an IL-13Rα2-based therapy(s) for anti-cancer stem cell activity involving i) administration of the therapy to a human with cancer, ii) monitoring cancer stem cells in or from the human prior to, during, and/or following therapy, and iii) determining whether the therapy resulted in a decrease in the amount of cancer stem cells.

### 5.2 TYPES OF CANCER

With any type of cancer for which a patient can be treated, the cancer stem cells thereof can be monitored in accordance with the methods described herein. The medical practitioner can diagnose the patient using any of the conventional cancer screening methods including, but not limited to physical examination (e.g., prostate examination, rectal examination, breast examination, lymph nodes examination, abdominal examination, skin surveillance, testicular exam, general palpation), visual methods (e.g., colonoscopy, bronchoscopy, endoscopy), PAP smear analyses (cervical cancer), stool guaiac analyses, blood tests (e.g., complete blood count (CBC) test, prostate specific antigen (PSA) test, carcinoembryonic antigen (CEA) test, cancer antigen (CA)-125 test, alpha-fetoprotein (AFP), liver function tests), karyotyping analyses, bone marrow analyses (e.g., in cases of hematological malignancies), histology, cytology, flow cytometry, a sputum analysis and imaging methods (e.g., computed tomography (CT), magnetic resonance imaging (MRI), ultrasound, X-ray imaging, mammography, PET scans, bone scans).

Non-limiting examples of cancers include: leukemias, such as but not limited to, acute leukemia, acute lymphocytic leukemia, acute myelocytic leukemias, such as, myeloblastic, promyelocytic, myelomonocytic, monocytic, and erythroleukemia leukemias and myelodysplastic syndrome (MDS); chronic leukemias, such as but not limited to, chronic myelocytic (granulocytic) leukemia, chronic lymphocytic leukemia, hairy cell leukemia; polycythemia vera; lymphomas such as but not limited to Hodgkin's disease, non-Hodgkin's disease; multiple myelomas such as but not limited to smoldering multiple myeloma, nonsecretory myeloma, osteosclerotic myeloma, plasma cell leukemia, solitary plasmacytoma and extramedullary plasmacytoma; Waldenström's macroglobulinemia; monoclonal gammopathy of undetermined significance; benign monoclonal gammopathy; heavy chain disease; bone and connective tissue sarcomas such as but not limited to bone sarcoma, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, synovial sarcoma; brain tumors such as but not limited to, glioma, astrocytoma, brain stem glioma, ependymoma, oligodendroglioma, nonglial tumor, acoustic neurinoma, craniopharyngioma, medulloblastoma, meningioma, pineocytoma, pineoblastoma, primary brain lymphoma; breast cancer including but not limited to ductal carcinoma, adenocarcinoma, lobular (small cell) carcinoma, intraductal carcinoma, medullary breast cancer, mucinous breast cancer, tubular breast cancer, papillary breast cancer, Paget's disease, and inflammatory breast cancer; adrenal cancer such as but not limited to pheochromocytom and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, vipoma, somatostatin-secreting tumor, and carcinoid or islet cell tumor; pituitary cancers such as but limited to Cushing's disease, prolactin-secreting tumor, acromegaly, and diabetes insipius; eye cancers such as but not limited to ocular melanoma such as iris melanoma, choroidal melanoma, and cilliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget's disease; cervical cancers such as but not limited to, squamous cell carcinoma, and adenocarcinoma; uterine cancers such as but not limited to endometrial carcinoma and uterine sarcoma; ovarian cancers such as but not limited to, ovarian epithelial carcinoma, borderline tumor, germ cell tumor, and stromal tumor; esophageal cancers such as but not limited to, squamous cancer, adenocarcinoma, adenoid cystic carcinoma, mucoepidermoid carcinoma, adenosquamous carcinoma, sarcoma, melanoma, plasmacytoma, verrucous carcinoma, and oat cell (small cell) carcinoma; stomach cancers such as but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers such as but not limited to hepatocellular carcinoma and hepatoblastoma; gallbladder cancers such as adenocarcinoma; cholangiocarcinomas such as but not limited to papillary, nodular, and diffuse; lung cancers such as non-small cell lung cancer, squamous cell carcinoma (epidermoid carcinoma), adenocarcinoma, large-cell carcinoma and small-cell lung cancer; testicular cancers such as but not limited to germinal tumor, seminoma, anaplastic, classic (typical), spermatocytic, nonseminoma, embryonal carcinoma, teratoma carcinoma, choriocarcinoma (yolk-sac tumor), prostate cancers such as but not limited to, prostatic intraepithelial neoplasia, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenoidcystic carcinoma; pharynx cancers such as but not limited to squamous cell cancer, and verrucous; skin cancers such as but not limited to, basal cell carcinoma, squamous cell carcinoma and melanoma, superficial spreading melanoma, nodular melanoma, lentigo malignant melanoma, acral lentiginous melanoma; kidney cancers such as but not limited to renal cell carcinoma, adenocarcinoma, hypernephroma, fibrosarcoma, transitional cell cancer (renal pelvis and/ or uterer); Wilms' tumor; bladder cancers such as but not limited to transitional cell carcinoma, squamous cell cancer, adenocarcinoma, carcinosarcoma. In addition, cancers include myxosarcoma, osteogenic sarcoma, endotheliosarcoma, lymphangioendotheliosarcoma, mesothelioma, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for a review of such disorders, *see* Fishman et al., 1985, Medicine, 2d Ed., J.B. Lippincott Co., Philadelphia and Murphy et al., 1997, Informed Decisions: The Complete Book of Cancer Diagnosis, Treatment, and Recovery, Viking Penguin, Penguin Books U.S.A., Inc., United States of America).

Other cancers or other abnormal proliferative diseases, include but are not limited to, the following: carcinoma, including that of the bladder, breast, colon, kidney, liver, lung, ovary, pancreas, stomach, cervix, thyroid and skin; including squamous cell carcinoma; hematopoietic tumors of lymphoid lineage, including leukemia, acute lymphocytic leukemia, acute lymphoblastic leukemia, B-cell lymphoma, T cell lymphoma, Burkitt's lymphoma; hematopoietic tumors of myeloid lineage, including acute and chronic myelogenous leukemias and promyelocytic leukemia; tumors of mesenchymal origin, including fibrosarcoma and rhabdomyoscarcoma; other tumors, including melanoma, seminoma, tetratocarcinoma, neuroblastoma and glioma; tumors of the central and peripheral nervous system, including astrocytoma, neuroblastoma, glioma, and schwannomas; tumors of mesenchymal origin, including fibrosarcoma, rhabdomyoscarama, and osteosarcoma; and other tumors, including melanoma, xeroderma pigmentosum, keratoactanthoma, seminoma, thyroid follicular cancer and teratocarcinoma. Cancers associated with aberrations in apoptosis are also included and are not be limited to, follicular lymphomas, carcinomas with p53 mutations, hormone dependent tumors of the breast, prostate and ovary, and precancerous lesions such as familial adenomatous polyposis, and myelodysplastic syndromes. Malignancy or dysproliferative changes (such as metaplasias and dysplasias), or hyperproliferative disorders of the skin, lung, liver, bone, brain, stomach, colon, breast, prostate, bladder, kidney, pancreas, ovary, and/or uterus are encompassed in the invention.

Non-limiting examples of leukemias and other blood-borne cancers include acute lymphoblastic leukemia "ALL", acute lymphoblastic B-cell leukemia, acute lymphoblastic T-cell leukemia, acute myeloblastic leukemia "AML", acute promyelocytic leukemia "APL", acute monoblastic leukemia, acute erythroleukemic leukemia, acute megakaryoblastic leukemia, acute myelomonocytic leukemia, acute nonlymphocyctic leukemia, acute undifferentiated leukemia, chronic myelocytic leukemia "CML", chronic lymphocytic leukemia "CLL", and hairy cell leukemia.

Non-limiting examples of lymphomas include Hodgkin's disease, non-Hodgkin's Lymphoma, Multiple myeloma, Waldenström's macroglobulinemia, Heavy chain disease, and Polycythemia vera.

Non-limiting examples of solid tumors encompassed in the invention include, but are not limited to fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, and retinoblastoma.

### 5.2.1 BRAIN CANCERS

In a specific embodiment, the methods described herein can be used in the prevention, treatment, and/or management of brain cancer. In certain embodiments, such methods comprise a step of monitoring the levels of cancer stem cells in a subject with brain cancer that has been treated in accordance with the methods described herein, e.g., the subject has been administered an EphA2-based cancer therapy, i.e., an EphA2 peptide or a compound (e.g., an antibody) that specifically targets EphA2 (e.g., targets EphA2 present on EphA2-expressing cancer stem cells).

The methods described herein can be used in the prevention, treatment, and/or management of brain cancer. In certain embodiments, such methods comprise a step of monitoring the levels of cancer stem cells in a subject with brain cancer that has been treated in accordance with the methods described herein, e.g., the subject has been administered an IL-13Rα2-based cancer therapy, i.e., an IL-13Rα2 peptide or a compound (e.g., an antibody) that specifically targets IL-13Rα2 (e.g., targets IL-13Rα2 present on IL-13Rα2-expressing cancer stem cells).

Any type of brain cancer can be treated in accordance with the methods described herein. Exemplary brain cancers include, but are not limited to, gliomas (including astrocytoma (e.g., pilocytic astrocytoma, diffuse astrocytoma, and anaplastic astrocytoma), glioblastoma, oligodendroglioma, brain stem glioma, non-brain stem glioma, ependymoma, and mixed tumors comprising more than one glial cell types), acoustic schwannoma, cranialpharyngioma, meningioma, medulloblastoma, primary central nervous system lymphoma, and tumors of the pineal (e.g., pineal astrocytic tumors and pineal parenchymal tumors) and pituitary glands. Gliomas additionally include recurrent malignant gliomas, high-risk WHO Grade II Astrocytomas, Oligo Astrocytomas, recurrent WHO Grade II Gliomas, newly-diagnosed malignant or intrinsic brain stem gliomas, incompletely resected non-brainstem gliomas, and recurrent unresectable low-grade gliomas. Additional types of brain cancer that can be treated in accordance with the methods described herein include adult low-grade infiltrative supratentorial astrocytoma/oligodendroglioma, adult low-grade infiltrative supratentorial astrocytoma, adult low-grade infiltrative supratentorial oligodendroglioma, adult low-grade infiltrative supratentorial astrocytoma/oligodendroglioma (excluding pilocytic astrocytoma), adult low-grade infiltrative supratentorial astrocytoma (excluding pilocytic astrocytoma), adult low-grade infiltrative supratentorial oligodendroglioma (excluding pilocytic astrocytoma), adult intracranial ependymoma, adult intracranial ependymoma (excluding subependymoma and myxopapillary), adult intracranial anaplastic ependymoma, anaplastic glioma, anaplastic glioblastoma, pilocytic astrocytoma, subependymoma, myxopapillary, 1 to 3 limited metastatic lesions (intraparenchymal), greater than 3 metastatic lesions (intraparenchymal), leptomeningeal metastases (neoplastic meningitis), primary CNS lymphoma, metastatic spine tumors, or meningiomas.

In one embodiment, the brain cancer treated in accordance with the methods described herein is a glioma. In a specific embodiment, the brain cancer treated in accordance with the methods described herein is recurrent malignant glioma. In another specific embodiment, the brain cancer treated in accordance with the methods described herein is recurrent WHO Grade II Glioma. In another specific embodiment, the brain cancer treated in accordance with the methods described herein is newly-diagnosed malignant or intrinsic brain stem glioma. In another specific embodiment, the brain cancer treated in accordance with the methods described herein is incompletely resected non-brainstem glioma. In another specific embodiment, the brain cancer treated in accordance with the methods described herein is recurrent unresectable low-grade glioma.

In one embodiment, a patient treated in accordance with the methods described herein is an adult with recurrent malignant glioma, recurrent glioblastoma, anaplastic astrocytoma, anaplastic oligodendroglioma, or anaplastic mixed oligoastrocytoma. The patient may be an adult with newly diagnosed high-risk low grade glioma. The patient may be an adult with newly diagnosed high-risk low grade astrocytoma. The patient may be an adult with newly diagnosed high-risk low grade oligoastrocytoma. The patient may be an adult with recurrent high-risk low grade astrocytoma. The patient may be an adult with recurrent high-risk low grade oligoastrocytoma. The patient may be an adult with recurrent high-risk low grade oligodendroglioma. The patient may be a child with newly diagnosed malignant glioma. In another embodiment, the patient is a child with intrinsic brain stem glioma. The patient may be a child with incompletely resected non-brainsteam high-grade glioma. The patient may be a child with recurrent unresectable low-grade glioma. The patient may be a child with newly diagnosed diffuse intrinsic pontine glioma. The patient may be a child with any high-grade glioma involving the brainstem and treated with RT or without chemotherapy during RT. In another embodiment, the patient is a child with newly diagnosed non-brainstem high-grade glioma treated with RT with chemotherapy. The patient may be a child with newly diagnosed non-brainstem high-grade glioma treated with RT without chemotherapy. The patient may be a child with recurrent non-brainstem high-grade glioma that has recurred after treatment.

In another embodiment, the brain cancer treated in accordance with the methods described herein is an astrocytoma. In a specific embodiment, the brain cancer treated in accordance with the methods described herein is high-risk WHO Grade II Astrocytoma. In another specific embodiment, the brain cancer treated in accordance with the methods described herein is Oligo Astrocytoma.

### 5.3 Peptides

### 5.3.1 Peptides derived from EphA2

EphA2 is a tyrosine kinase receptor that is involved in the formation of the notochord via interaction with ephrinA1. (see, e.g., Naruse-Nakajima et al., Mech. Dev., 102: 95-105, 2001).

Any EphA2 peptide capable of serving as an HLA-A2 restricted cytotoxic T lymphocyte (CTL) epitope may be used in accordance with the described herein. In some embodiments, the EphA2 peptide used in a vaccine described herein comprises SEQ ID NO: 1. In some embodiments, the EphA2 peptide used in a vaccine described herein consists of SEQ ID NO:1.

The EphA2 peptide used in accordance with the methods described herein may comprise a mutated version of an EphA2 peptide, e.g., a mutated version of SEQ ID NO: 1, wherein the mutated version comprises at least 1, at least 2, or at least 3 amino acid subsitutions (e.g., conservative substitutions), additions, or deletions.

The EphA2 peptide used in accordance with the methods described herein may comprise an amino acid sequence with at least 50%, 60%, 70%, 80%, or 90% identity to SEQ ID NO: 1. The EphA2 peptide used in accordance with the methods described herein may comprise an amino acid sequence with at least 50% to 60%, 50% to 70%, 60% to 70%, 70% to 80%, 70% to 90%, or 80% to 90% identity to SEQ ID NO: 1. The EphA2 peptide used in accordance with the methods described herein may comprise an amino acid sequence with at least 50%, 60%, 70%, 80%, or 90% similarity to SEQ ID NO:1. The EphA2 peptide used in accordance with the methods
described herein may comprise an amino acid sequence with at least 50% to 60%, 50% to 70%, 60% to 70%, 70% to 80%, 70% to 90%, or 80% to 90% similarity to SEQ ID NO:1. Alternatively, the EphA2 peptide used in accordance with the methods described herein does not comprise or consist of SEQ ID NO:1, i.e., the EphA2 peptide is derived from a different portion of EphA2 than is SEQ ID NO: 1.

### 5.3.2 Peptides derived from IL-13Rα2

IL-13Rα2 a membrane glycoprotein that binds as a component of a heterodimer to the Th2 cytokine, IL-13, which induces monocytes and macrophages to produce TGFβ (see, e.g., Fichtner-Feigl et al., Nat. Med., 12: 99-106, 2006).

Any IL-13Rα2 peptide capable of serving as an HLA-A2 restricted cytotoxic T lymphocyte (CTL) epitope may be used in a vaccine described herein. In some embodiments, the IL-13Rα2 peptide used in accordance with the methods described herein comprises any one of SEQ ID NOs:2-5.

The IL-13Rα2 peptide used in a vaccine described herein may comprise a mutated version of SEQ ID NO:2, wherein the mutated version of SEQ ID NO:2 comprises at least 1, at least 2, or at least 3 amino acid subsitutions (e.g., conservative substitutions), additions, or deletions.

The IL-13Rα2 peptide used in a vaccine described herein may comprise an amino acid sequence with at least 50%, 60%, 70%, 80%, or 90% identity to SEQ ID NO:2. The IL-13Rα2 peptide used in a vaccine described herein may comprise an amino acid sequence with at least 50% to 60%, 50% to 70%, 60% to 70%, 70% to 80%, 70% to 90%, or 80% to 90% identity to SEQ ID NO:2. The IL-13Rα2 peptide used in a vaccine described herein comprises an amino acid sequence with at least 50%, 60%, 70%, 80%, or 90% similarity to SEQ ID NO:2. The IL-13Rα2 peptide used in a vaccine described herein may comprise an amino acid sequence with at least 50% to 60%, 50% to 70%, 60% to 70%, 70% to 80%, 70% to 90%, or 80% to 90% similarity to SEQ ID NO:2.

### 5.4 Immune Response Modifiers

The EphA2 and/or IL-13Rα2 peptides provided herein and compositions thereof may be administered concurrently with an immune response modifier. Immune response modifiers include agents capable of modifying the immune response of a subject. An immune response modifier may polarize the immune response of a subject toward a Th1 response. An immune response modifier may polarize the immune response of a subject toward a Th2 response. The immune response modifier may bind to a toll-like receptor (TLR) such as TLR3. Exemplary immune response modifiers that may be administered concurrently with the EphA2 and/or IL-13Rα2 peptides provided herein include, without limitation, Polyinosinic-Polycytidylic acid stabilized with polylysine and carboxymethylcellulose (poly-ICLC; also known as Hiltonol), imiquimod (Aldara^{®}; Beselna^{®}), and MIS-416 (Innate Therapeutics).

### 5.5 Adjuvants

The EphA2 and/or IL-13Rα2 peptides provided herein may be administered concurrently with an adjuvant. The term "adjuvant" refers to an agent that when administered concurrently with or in the same composition as an EphA2 and/or IL-13Rα2 peptide augments, accelerates, prolongs, enhances and/or boosts the immune response to the ILEphA2 and/or IL-13Rα2 peptide. The adjuvant may generate an immune response to the EphA2 and/or IL-13Rα2 peptide and does not produce an allergy or other adverse reaction. Adjuvants can enhance an immune response by several mechanisms including, e.g., lymphocyte recruitment, stimulation of B and/or T cells, stimulation of dendritic cells and stimulation of macrophages.

Specific examples of adjuvants include, but are not limited to, Montanide ISA-51, Montanide ISA 50V, Montanide, ISA 206, Montanide IMS 1312, VaxImmune^{®} (CpG7909; Coley Pharmaceuticals), aluminum salts (alum) (such as aluminum hydroxide, aluminum phosphate, and aluminum sulfate), 3 De-O-acylated monophosphoryl lipid A (MPL) (see GB 2220211), MF59 (Novartis), AS03 (GlaxoSmithKline), AS04 (GlaxoSmithKline), polysorbate 80 (Tween 80; ICL Americas, Inc.), imidazopyridine compounds (see International Application No. PCT/US2007/064857, published as International Publication No. WO2007/109812), imidazoquinoxaline compounds (see International Application No. PCT/US2007/064858, published as International Publication No. WO2007/109813) and saponins, such as QS21 (see Kensil et al., in Vaccine Design: The Subunit and Adjuvant Approach (eds. Powell & Newman, Plenum Press, NY, 1995); U.S. Pat. No. 5,057,540). The adjuvant may be
Freund's adjuvant (complete or incomplete). Other adjuvants are oil in water emulsions (such as squalene or peanut oil), optionally in combination with immune stimulants, such as monophosphoryl lipid A (see Stoute etal., N. Engl. J. Med. 336, 86-91 (1997)). Another adjuvant is CpG (Bioworld Today, Nov. 15, 1998). Such adjuvants can be used with or without other specific immunostimulating agents such as MPL or 3-DMP, QS21, polymeric or monomeric amino acids such as polyglutamic acid or polylysine, or other immunopotentiating agents. It should be understood that different formulations of EphA2 peptides may comprise different adjuvants or may comprise the same adjuvant.

### 5.6 Helper T Cell Epitopes

The EphA2 and/or IL-13Rα2 peptides provided herein may be administered concurrently with a helper T cell epitope. Helper T cell epitopes include agents that are capable of inducing a helper T cell response by the immune system. Helper T cells are CD4+ T cells. Helper T cell epitopes are presented by Class II MHC molecules, and may be recognized by the T cell receptor (TCR) of helper T cells (CD4+ T cells), thereby activating the CD4+ T cells, causing them to proliferate, secrete cytokines such as IL2, and activate professional antigen presenting cells. Through a variety of mechanisms, activated helper T cells also stimulate killer T cells (also known as CD8+ T cells), thereby prolonging and increasing the CD8+ T cell response. Exemplary helper T cell epitopes that can be administered concurrently with the EphA2 peptides provided herein include, without limitation, PADRE (see, e.g., Alexander et al, Immunity, 1:751-761, 1994), HBVcore₁₂₈₋₁₄₀, and tetanus toxoid.

### 5.7 Production and Purification of epha2 Peptides

The EphA2 and/or IL-13Rα2 peptides described herein can be made by standard recombinant DNA techniques or by protein synthetic techniques, e.g., by use of a peptide synthesizer. For example, a nucleic acid molecule encoding an EphA2 and/or IL-13Rα2 peptide can be synthesized by conventional techniques including automated DNA synthesizers. As another example, the EphA2 and/or IL-13Rα2 peptides described herein may be generated using conventional step-wise solution or solid phase synthesis (see, e.g., Chemical Approaches to the Synthesis of Peptides and Proteins, Williams et al., Eds., 1997, CRC Press, Boca Raton Fla., and references cited therein; Solid Phase Peptide Synthesis: A Practical Approach, Atherton & Sheppard, Eds., 1989, IRL Press, Oxford, England, and references cited therein) or through the use of segment condensation (see, e.g., Liu et al., 1996, Tetrahedron Lett. 37(7):933-936; Baca, et al., 1995, J. Am. Chem. Soc. 117:1881-1887; Tam et al., 1995, Int. J. Peptide Protein Res. 45:209-216; Schnolzer and Kent, 1992, Science 256:221-225; Liu and Tam, 1994, J. Am. Chem. Soc. 116(10):4149-4153; Liu and Tam, 1994, Proc. Natl. Acad. Sci. USA 91:6584-6588; Yamashiro and Li, 1988, Int. J. Peptide Protein Res. 31:322-334).

The EphA2 or IL-13Rα2 peptides described herein may be obtained from any information available to those of skill in the art (*i.e.*, from Genbank, the literature, or by routine cloning). A nucleotide sequence coding for an EphA2 or IL-13Rα2 peptide can be inserted into an appropriate expression vector, *i.e.*, a vector which contains the necessary elements for the transcription and translation of the inserted protein-coding sequence. A variety of host-vector systems may be utilized to express the protein-coding sequence. These include but are not limited to mammalian cell systems infected with virus (*e.g*., vaccinia virus, adenovirus, etc.); insect cell systems infected with virus (*e.g.*, baculovirus); microorganisms such as yeast (*e.g. Pichia*) containing yeast vectors; or bacteria (such as *E*. *coli*) transformed with bacteriophage, DNA, plasmid DNA, or cosmid DNA. The expression elements of vectors vary in their strengths and specificities. Depending on the host-vector system utilized, any one of a number of suitable transcription and translation elements may be used. The peptide may be expressed in *E. coli.* The peptide may be expressed in *Pichia.*

Once an EphA2 or IL-13Rα2 peptide has been produced by recombinant expression or by chemical synthesis, it may be purified by any method known in the art for purification of a protein, for example, by chromatography (*e.g*., ion exchange, affinity, particularly by affinity for the specific antigen after Protein A, and sizing column chromatography), centrifugation, differential solubility, or by any other standard technique for the purification of proteins.

### 5.8 PHARMACEUTICAL COMPOSITIONS AND ROUTES OF ADMINISTRATION

Provided herein are pharmaceutical compositions comprising EphA2 and/or IL-13Rα2 eptides for use in the methods described herein. A composition provided herein comprises EphA2 and/or IL-13Rα2 peptide and one or more additional peptides or agents. The compositions provided herein may comprise an EphA2 and/or IL-13Rα2 peptide and a helper T cell epitope, an adjuvant, and/or an immune response modifier. The pharmaceutical compositions provided herein are suitable for veterinary and/or human administration.

The pharmaceutical compositions provided herein can be in any form that allows for the composition to be administered to a subject, said subject preferably being an animal, including, but not limited to a human, mammal, or non-human animal, such as a cow, horse, sheep, pig, fowl, cat, dog, mouse, rat, rabbit, guinea pig, etc., and is more preferably a mammal, and most preferably a human.

The compositions provided herein may be in the form of a liquid (e.g., an elixir, syrup, solution, emulsion, or suspension). Typical routes of administration of the liquid compositions provided herein may include, without limitation, parenteral, intradermal, intratumoral, intracerebral, and intrathecal. Parenteral administration includes, without limitation, subcutaneous, intranodal, intravenous, intramuscular, intraperitoneal, and intrapleural administration techniques. The compositions may be administered parenterally. In a composition for administration by injection, one or more of a surfactant, preservative, wetting agent, dispersing agent, suspending agent, buffer, stabilizer, and isotonic agent may be included. A pump may be used to deliver the vaccines (see, e.g, Sefton, CRC Crit. Ref. Biomed. Eng. 1987, 14, 201; Buchwald et al., Surgery 1980, 88: 507; Saudek et al., N. Engl. J. Med. 1989, 321: 574). The pump may be, but is not limited to, an insulin-like pump.

Materials used in preparing the pharmaceutical compositions provided herein can be non-toxic in the amounts used. It may be evident to those of ordinary skill in the art that the optimal dosage of the active ingredient(s) in the pharmaceutical composition will depend on a variety of factors. Relevant factors include, without limitation, the type of subject (e.g., human), the overall health of the subject, the type of cancer the subject is in need of treatment of, the use of the composition as part of a multi-drug regimen, the particular form of the peptide being administered, the manner of administration, and the composition employed.

The liquid compositions provided herein, whether they are solutions, suspensions, or other like form, can also include one or more of the following: sterile diluents such as water for injection, saline solution, preferably physiological saline, Ringer's solution, isotonic sodium chloride, fixed oils such as synthetic mono or digylcerides which can serve as the solvent or suspending medium, polyethylene glycols, glycerin, cyclodextrin, propylene glycol, or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates, or phosphates; and agents for the adjustment of tonicity such as sodium chloride or dextrose. A parenteral composition can be enclosed in an ampoule, a disposable syringe, or a multiple-dose vial made of glass, plastic or other material. An injectable composition is preferably sterile.

The compositions provided herein may comprise a pharmaceutically acceptable carrier or vehicle. As used herein, the term "pharmaceutically acceptable" means approved by a regulatory agency of the Federal or a state government or listed in the U.S. Pharmacopeia or other generally recognized pharmacopeiae for use in animals, and more particularly in humans. The term "carrier" refers to a diluent, adjuvant, excipient, or vehicle with which the pharmaceutical composition is administered. Saline solutions and aqueous dextrose and glycerol solutions can also be employed as liquid carriers, particularly for injectable solutions. Suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Examples of suitable pharmaceutical carriers are described in "Remington's Pharmaceutical Sciences" by E.W. Martin. The formulation should suit the mode of administration.

The compositions provided herein may be formulated in accordance with routine procedures as a pharmaceutical composition adapted for parenteral administration to animals, particularly human beings. Generally, the ingredients in the compositions are supplied either separately or mixed together in unit dosage form, for example, as a dry lyophilized powder or water free concentrate in a hermetically sealed container such as an ampoule or sachet indicating the quantity of active agent. Where a composition described herein is administered by injection, an ampoule of sterile water for injection or saline can be provided so that the ingredients can be mixed prior to administration, if necessary.

The compositions described herein can comprise an additional active agent selected from among those including, but not limited to, an additional prophylactic agent, an additional therapeutic agent, an antiemetic agent, a hematopoietic colony stimulating factor, an adjuvant therapy, an antibody/antibody fragment-based agent, an anti-depressant and an analgesic agent. The additional active agent may be a second EphA2 and/or IL-13Rα2 peptide (i.e., an EphA2 and/or IL-13Rα2 peptide different from the one that forms the base of the composition). The additional active agent may be a second peptide that is not an EphA2 and/or IL-13Rα2 peptide.

The pharmaceutical compositions provided herein can be prepared using methodology well known in the pharmaceutical art. For example, a composition intended to be administered by injection can be prepared by combining the EphA2 and/or IL-13Rα2 peptides described herein with water and/or other liquid components so as to form a solution. A surfactant can be added to facilitate the formation of a homogeneous solution or suspension.

The pharmaceutical compositions described herein can be included in a container, pack, or dispenser together with instructions for administration.

### 5.8.1 DOSAGE AND FREQUENCY OF ADMINISTRATION

The amount of a composition described herein (e.g., a composition comprising an EphA2 and/or IL-13Rα2 peptide; a composition comprising an EphA2 peptide and an IL-13Rα2 peptide a composition comprising an EphA2 and/or IL-13Rα2 peptide and a helper T cell epitope, an adjuvant) which will be effective in the treatment, prevention, and or management of cancer may depend on the status of the cancer, the patient to whom the composition(s) is to be administered, the route of administration, and/or the type of cancer. Such doses can be determined by standard clinical techniques and may be decided according to the judgment of the practitioner.

For example, effective doses may vary depending upon means of administration, target site, physiological state of the patient (including age, body weight, health), whether the patient is human or an animal, other medications administered, and whether treatment is prophylactic or therapeutic. Usually, the patient is a human but nonhuman mammals including transgenic mammals can also be treated. Treatment dosages are optimally titrated to optimize safety and efficacy.

An *in vitro* assay may be employed to help identify optimal dosage ranges. Effective doses may be extrapolated from dose response curves derived from in vitro or animal model test systems.

A composition may comprise about 25, 50, 75, 100, 125, 150, 175, 200, 225, 250, 275, 300, 325, 350, 375, 400, 425, 450, 475, 500, 550, 600, 650, 700, 750, or 800 µg of an EphA2 peptide per dose. In other cases, compositions comprise about 25 to 50, 25 to 75, 25 to 100, 50 to 100, 50 to 150, 50 to 200, 100 to 150, 100 to 200, 100 to 250, 100 to 300, 150 to 200, 150 to 250, 150 to 300, 200 to 250, 250 to 300, 250 to 350, 250 to 400, 300 to 350, 300 to 400, 300 to 450, 300 to 500, 350 to 400, 350 to 450, 400 to 500, 400 to 600, 500 to 600, 500 to 700, 600 to 700, 600 to 800, or 700 to 800 µg of an EphA2 and/or IL-13Rα2 peptide per dose. In other cases, compositions comprise about 5 µg to 100 mg, 15 µg to 50 mg, 15 µg to 25 mg, 15 µg to 10 mg, 15 µg to 5 mg, 15 µg to 1 mg, 15 µg to 100 µg, 15 µg to 75 µg, 5 µg to 50 µg, 10 µg to 50 µg, 15 µg to 45 µg, 20 µg to 40 µg, or 25 to 35 µg of an EphA2 and/or IL-13Rα2 peptide per kilogram of the patient.

Compositions comprising an EphA2 and/or IL-13Rα2 peptide may be administered concurrently with a helper T cell epitope. Such compositions may be administered concurrently with about 25, 50, 75, 100, 125, 150, 175, 200, 225, 250, 275, 300, 325, 350, 375, 400, 425, 450, 475, 500, 550, or 600 µg of a helper T cell epitope. Such compositions may be administered concurrently with about 25 to 50, 25 to 75, 25 to 100, 50 to 100, 50 to 150, 50 to 200, 100 to 150, 100 to 200, 100 to 250, 100 to 300, 150 to 200, 150 to 250, 150 to 300, 200 to 250, 250 to 300, 250 to 350, 250 to 400, 300 to 350, 300 to 400, 300 to 450, 300 to 500, 350 to 400, 350 to 450, 400 to 500, 400 to 600, or 500 to 600 µg of a helper T cell epitope.

The compositions comprising an EphA2 and/or IL-13Rα2 peptide may be administered concurrently with an immune response modifier, e.g., about 100, 200, 300, 400, 500, 600, 700, 800, 900, 1000, 1100, 1200, 1300, 1400, 1500, 1600, 1700, or 1800 µg of an immune response modifier; or about 100 to 300, 200 to 400, 400 to 800, 600 to 800, 800 to 1000, 800 to 1200, 1000 to 1200, 1000 to 1400, 1200 to 1400, 1200 to 1600, 1400 to 1600, 1400 to 1800, or 1600 to 1800 µg of an immune response modifier.

The compositions comprising an EphA2 and/or IL-13Rα2 peptide may be administered concurrently with an adjuvant. The compositions comprising an EphA2 peptide may be mixed 0.5 to 1, 1 to 0.5, 1 to 1, 1 to 2, 1 to 3, 2 to 1, or 3 to 1 with an adjuvant.

A composition described herein may be administered to a subject once as a single dose. A composition described herein may be administered in multiple doses (e.g., 1, 2, 3, 4, 5, 6, 7, 8, 9, 10, or more than 10 doses), wherein the doses may be separated by at least 1 day, 2 days, 3 days, 4, days 5 days, 6 days, 7 days, 8 days, 9 days, 10 days, 15 days, or 30 days.

A composition described herein may be administered over the course of 21 weeks, with administrations occurring on weeks 0, 3, 6, 9, 12, 15, 18 and 21. The composition may be administered concurrently with a helper T cell epitope, an adjuvant, and/or an immune response modifer. A composition described herein may be administered over the course of 21 weeks, with administrations occurring on weeks 0, 3, 6, 9, 12, 15, 18 and 21, and the composition is administered concurrently with an immune response modifier, wherein the immune response modifier is administered on the day of each administration of the composition comprising an EphA2 and/or IL-13Rα2 peptide and on day 4 after each administration of the composition comprising an EphA2 and/or IL-13Rα2 peptide. A composition described herein may be administered over the course of 21 weeks, with administrations occurring on weeks 0, 3, 6, 9, 12, 15, 18 and 21, and the composition is administered concurrently with an immune response modifier, wherein the immune response modifier is administered on the day of each administration of the composition comprising an EphA2 and/or IL-13Rα2 peptide.

### 5.8.2 PATIENT POPULATIONS

An EphA2 and/or IL-13Rα2 peptide or composition thereof may be administered to a naive subject, *i.e.,* a subject that does not have cancer. In one embodiment, an EphA2 and IL-13Rα2 peptide or composition thereof is administered to a naive subject that is at risk of acquiring cancer.

In certain embodiments, an EphA2 and IL-13Rα2 peptide or composition thereof is administered to a patient who has been diagnosed with cancer. In some embodiments, an EphA2 and/or IL-13Rα2 peptide or composition thereof is administered to a patient with cancer before symptoms manifest or symptoms become severe. In a specific embodiment, the cancer is brain cancer.

In certain embodiments, an EphA2 and IL-13Rα2 peptide or composition thereof is administered to a patient who is in need of treatment, prevention, and/or management of cancer. Such subjects may or may not have been previously treated for cancer or may be in remission, relapsed, or may have failed treatment. Such patients may also have abnormal cytogenetics.

The subject may have been diagnosed with cancer using techniques known to one of skill in the art including, but not limited to, neurological examination; imaging methods (e.g., computed tomography (CT), magnetic resonance imaging (MRI), ultrasound, X-ray imaging, fluid-attenuated inversion-recovery (FLAIR) sequences, T2 weighted imaging, and positron emission tomography (PET) scans); and biopsy (e.g., sterotactic biopsy). Tumor response to therapy may be evaluated by McDonald criteria or Response assesment in neuro-oncology (RANO) criteria. Tumor size or response to treatment can be evaluated by various magnetic resonance imaging techniques including diffusion-weighted imaging, perfusion-weighted imaging, dynamic contrast-enhanced T1 permeability imaging, dynamic susceptibility contrast, diffusion-tensor imaging, and magnetic resonance spectroscopy, anatomic MRI T2-weighted images, fluid attenuated inversion recovery (FLAIR) T2-weighted images, and gadolinium-enhanced T1-weighted images. These imagining techniques can be used to assess tumor cellularity, white matter invasion, metabolic derangement including hypoxia and necrosis, neovascular capillary blood volume, or permeability. Positron emission tomograph (PET) technology can also be used to image tumor response, such as 18F-fluoromisonidazole PET and 3'-deoxy-3'-18F-flurorothymidine PET.

In some embodiments, an EphA2 and IL-13Rα2 peptide or composition thereof is administered to a subject that is in remission from brain cancer. In a specific embodiment, the subject has no detectable brain cancer, *i.e.*, no brain cancer is detectable using a conventional method described herein (*e.g.*, MRI) or known to one of skill in the art.

In one embodiment, an EphA2 and IL-13Rα2 peptide or composition thereof is administered to a subject diagnosed with glioma. In a specific embodiment, an EphA2 and IL-13Rα2 peptide or composition thereof is administered to a subject diagnosed with astrocytoma (e.g., pilocytic astrocytoma, diffuse astrocytoma, and anaplastic astrocytoma). In another specific embodiment, an EphA2 and IL-13Rα2 peptide or composition thereof is administered to a subject diagnosed with glioblastoma. In another specific embodiment, an EphA2 and IL-13Rα2 peptide or composition thereof is administered to a subject diagnosed with oligodendroglioma. In another specific embodiment, an EphA2 and IL-13Rα2 peptide or composition thereof is administered to a subject diagnosed with brain stem glioma. In another specific embodiment, vis administered to a subject diagnosed with ependymoma. In another specific embodiment, an EphA2 and IL-13Rα2 peptide or composition thereof is administered to a subject diagnosed with a mixed tumor comprising more than one glial cell types.

In a specific embodiment, an EphA2 and IL-13Rα2 peptide or composition thereof is administered to a subject diagnosed with recurrent malignant glioma. In another specific embodiment, an EphA2 and IL-13Rα2 peptide or composition thereof is administered to a subject diagnosed with high-risk WHO Grade II Astrocytomas. In another specific embodiment, an EphA2 and IL-13Rα2 peptide or composition thereof is administered to a subject diagnosed with Oligo Astrocytoma. In another specific embodiment, an EphA2 and IL-13Rα2 peptide or composition thereof is administered to a subject diagnosed with recurrent WHO Grade II Glioma. In another specific embodiment, an EphA2 and IL-13Rα2 peptide or composition thereof is administered to a subject diagnosed with newly-diagnosed malignant or intrinsic brain stem glioma. In another specific embodiment, an EphA2 and IL-13Rα2 peptide or composition thereof is administered to a subject diagnosed with incompletely resected non-brainstem glioma. In another specific embodiment, an EphA2 and IL-13Rα2 peptide or composition thereof is administered to a subject diagnosed with recurrent unresectable low-grade glioma.

In a specific embodiment, an EphA2 and IL-13Rα2 peptide or composition thereof is administered to a subject diagnosed with acoustic schwannoma. In another specific embodiment, an EphA2 and IL-13Rα2 peptide or composition thereof is administered to a subject diagnosed with cranial pharyngioma. In another specific embodiment, an EphA2 and IL-13Rα2 peptide or composition thereof is administered to a subject diagnosed with meningioma. In another specific embodiment, an EphA2 and IL-13Rα2 peptide or composition thereof is administered to a subject diagnosed with medulloblastoma. In another specific embodiment, an EphA2 and IL-13Rα2 peptide or composition thereof described herein is administered to a subject diagnosed with primary central nervous system lymphoma. In another specific embodiment, an EphA2 and IL-13Rα2 peptide or composition thereof is administered to a subject diagnosed with a tumor of the pineal gland (e.g., a pineal astrocytic tumor or a pineal parenchymal tumor). In another specific embodiment, an EphA2 and IL-13Rα2 peptide or composition thereof is administered to a subject diagnosed with a tumor of the pituitary gland.

### 5.8.3 COMBINATION THERAPIES

The methods provided herein for preventing, treating, and/or managing cancer comprise administering to a patient (*e.g.*, a human patient) in need thereof a prophylactically and/or a therapeutically effective regimen, the regimen comprising administering to the patient EphA2 and/or IL-13Rα2 peptide or composition thereof described herein and one or more additional therapies. An EphA2 peptide or composition thereof described herein and an additional therapy can be administered separately, concurrently, or sequentially. The combination therapies can act additively or synergistically. A combination therapy provided herein comprises an EphA2 peptide and an IL-13Rα2.

The combination therapies can be administered to a subject in the same pharmaceutical composition. Alternatively, the combination therapies can be administered concurrently to a subject in separate pharmaceutical compositions. The combination therapies may be administered to a subject by the same or different routes of administration.

Any therapy (e.g., therapeutic or prophylactic agent) which is useful, has been used, or is currently being used for the prevention, treatment, and/or management of cancer (e.g., brain cancer) can be used in combination with an EphA2 and/or IL-13Rα2 peptide or composition described herein in the methods described herein. Therapies include, but are not limited to, peptides, polypeptides, antibodies, conjugates, nucleic acid molecules, small molecules, mimetic agents, synthetic drugs, inorganic molecules, and organic molecules. Non-limiting examples of cancer therapies include chemotherapy, radiation therapy, hormonal therapy, surgery, small molecule therapy, anti-angiogenic therapy, differentiation therapy, epigenetic therapy, radioimmunotherapy, targeted therapy, and/or biological therapy including immunotherapy. In certain embodiments, a prophylactically and/or therapeutically effective regimen of the invention comprises the administration of a combination of therapies.

Examples of cancer therapies which can be used in combination with EphA2 and/or IL-13Rα2 peptide or composition thereof described herein in accordance with the methods described herein include, but are not limited to: acivicin; aclarubicin; acodazole hydrochloride; acronine; adozelesin; aldesleukin; altretamine; ambomycin; ametantrone acetate; aminoglutethimide; amsacrine; anastrozole; anthracyclin; anthramycin; asparaginase; asperlin; azacitidine (Vida*za*); azetepa; azotomycin; batimastat; benzodepa; bicalutamide; bisantrene hydrochloride; bisnafide dimesylate; bisphosphonates (*e.g*., pamidronate (Aredria), sodium clondronate (Bonefos), zoledronic acid (Zometa), alendronate (Fosamax), etidronate, ibandornate, cimadronate, risedromate, and tiludromate); bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine (Ara-C); dacarbazine; dactinomycin; daunorubicin hydrochloride; decitabine (Dacogen); demethylation agents, dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; EphA2 inhibitors; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; histone deacetylase inhibitors (HDACs) gemcitabine hydrochloride; hydroxyurea; idarubicin hydrochloride; ifosfamide; ilmofosine; imatinib mesylate (Gleevec, Glivec); interleukin II (including recombinant interleukin II, or rIL2), interferon alpha-2a; interferon alpha-2b; interferon alpha-n1 ; interferon alpha-n3; interferon beta-I a; interferon gamma-I b; iproplatin; irinotecan hydrochloride; lanreotide acetate; lenalidomide (Revlimid); letrozole; leuprolide acetate; liarozole hydrochloride; lometrexol sodium; lomustine; losoxantrone hydrochloride; masoprocol; maytansine; mechlorethamine hydrochloride; anti-CD2 antibodies (*e.g.*, siplizumab (MedImmune Inc.; International Publication No. WO 02/098370); megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxaliplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride.

Other examples of cancer therapies which can be used in combination with an EphA2 and/or IL-13Rα2 peptide or composition thereof described herein in accordance with the methods described herein include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; HMG CoA reductase inhibitors (e.g., atorvastatin, cerivastatin, fluvastatin, lescol, lupitor, lovastatin, rosuvastatin, and simvastatin); hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4- iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; LFA-3TIP (Biogen, Cambridge, MA; International Publication No. WO 93/0686 and U.S. Patent No. 6,162,432); liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; 5-fluorouracil; leucovorin; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; thalidomide; velaresol; veramine; verdins; verteporfin; vinorelbine; vinxaltine; VITAXIN^{™} (see U.S. Patent Pub. No. US 2002/0168360 A1, dated November 14, 2002, entitled "Methods of Preventing or Treating Inflammatory or Autoimmune Disorders by Administering Integrin αvβ3 Antagonists in Combination With Other Prophylactic or Therapeutic Agents"); vorozole; zanoterone; zeniplatin; zilascorb; and zinostatin stimalamer.

The therapy(ies) used in combination with an EphA2 and/or IL-13Rα2 peptide or composition thereof described herein in accordance with the methods described herein may be an immunomodulatory agent. Non-limiting examples of immunomodulatory agents include proteinaceous agents such as cytokines, peptide mimetics, and antibodies (e.g., human, humanized, chimeric, monoclonal, polyclonal, Fvs, ScFvs, Fab or F(ab)2 fragments or epitope binding fragments), nucleic acid molecules (e.g., antisense nucleic acid molecules and triple helices), small molecules, organic compounds, and inorganic compounds. In particular, immunomodulatory agents include, but are not limited to, methotrexate, leflunomide, cyclophosphamide, cytoxan, Immuran, cyclosporine A, minocycline, azathioprine, antibiotics *(e.g.,* FK506 (tacrolimus)), methylprednisolone (MP), corticosteroids, steroids, mycophenolate mofetil, rapamycin (sirolimus), mizoribine, deoxyspergualin, brequinar, malononitriloamindes (e.g., leflunamide), T cell receptor modulators, cytokine receptor modulators, and modulators mast cell modulators. Other examples of immunomodulatory agents can be found, *e.g.,* in U.S. Publication No. 2005/0002934 A1 at paragraphs 259-275. The immunomodulatory agent may be a chemotherapeutic agent. Alternatively, the immunomodulatory agent is an immunomodulatory agent other than a chemotherapeutic agent. Alternatively, the therapy(ies) used in accordance with the invention is not an immunomodulatory agent.

The therapy(ies) used in combination with an EphA2 and/or IL-13Rα2 peptide or composition thereof described herein in accordance with the methods described herein may be an anti-angiogenic agent. Non-limiting examples of anti-angiogenic agents include proteins, polypeptides, peptides, conjugates, antibodies (e.g., human, humanized, chimeric, monoclonal, polyclonal, Fvs, ScFvs, Fab fragments, F(ab)2 fragments, and antigen-binding fragments thereof) such as antibodies that specifically bind to TNF-α, nucleic acid molecules (e.g., antisense molecules or triple helices), organic molecules, inorganic molecules, and small molecules that reduce or inhibit angiogenesis. Other examples of anti-angiogenic agents can be found, *e.g.,* in U.S. Publication No. 2005/0002934 A1 at paragraphs 277-282. The anti-angiogenic therapy may be bevacizumab (Avastin^{®}). Alternatively, the therapy(ies) used in accordance with the invention is not an anti-angiogenic agent.

The therapy(ies) used in combination with an EphA2 and/or IL-13Rα2 peptide or composition thereof described herein in accordance with the methods described herein may be an anti-inflammatory agent. Non-limiting examples of anti-inflammatory agents include any anti-inflammatory agent, including agents useful in therapies for inflammatory disorders, well-known to one of skill in the art. Non-limiting examples of anti-inflammatory agents include non-steroidal anti-inflammatory drugs (NSAIDs), steroidal anti-inflammatory drugs, anticholinergics (e.g., atropine sulfate, atropine methylnitrate, and ipratropium bromide (ATROVENT^{™})), beta2-agonists (e.g., abuterol (VENTOLIN^{™} and PROVENTIL^{™}), bitolterol (TORNALATE^{™}), levalbuterol (XOPONEX^{™}), metaproterenol (ALUPENT^{™}), pirbuterol (MAXAIR^{™}), terbutlaine (BRETHAIRE^{™} and BRETHINE^{™}), albuterol (PROVENTIL^{™}, REPETABS^{™}, and VOLMAX^{™}), formoterol (FORADII, AEROLIZER^{™}), and salmeterol (SEREVENT^{™} and SEREVENT DISKUS^{™})), and methylxanthines (e.g., theophylline (UNIPHYL^{™}, THEO-DUR^{™}, SLO-BID^{™}, AND TEHO-42^{™})). Examples of NSAIDs include, but are not limited to, aspirin, ibuprofen, celecoxib (CELEBREX^{™}), diclofenac (VOLTAREN^{™}), etodolac (LODINE^{™}), fenoprofen (NALFON^{™}), indomethacin (INDOCIN^{™}), ketoralac (TORADOL^{™}), oxaprozin (DAYPRO^{™}), nabumentone (RELAFEN^{™}), sulindac (CLINORIL^{™}), tolmentin (TOLECTIN^{™}), rofecoxib (VIOXX^{™}), naproxen (ALEVE^{™}, NAPROSYN^{™}), ketoprofen (ACTRON^{™}) and nabumetone (RELAFEN^{™}). Such NSAIDs function by inhibiting a cyclooxgenase enzyme (e.g., COX-1 and/or COX-2). Examples of steroidal anti-inflammatory drugs include, but are not limited to, glucocorticoids, dexamethasone (DECADRON^{™}), corticosteroids (e.g., methylprednisolone (MEDROL^{™})), cortisone, hydrocortisone, prednisone (PREDNISONE^{™} and DELTASONE^{™}), prednisolone (PRELONE^{™} and PEDIAPRED^{™}), triamcinolone, azulfidine, and inhibitors of eicosanoids (*e.g.*, prostaglandins, thromboxanes, and leukotrienes. Other examples of anti-inflammatory agents can be found, *e.g.,* in U.S. Publication No. 005/0002934 A1 at paragraphs 290-294. Alternatively, the therapy(ies) used in accordance with the invention is not an anti-inflammatory agent.

The therapy(ies) used in combination with an EphA2 and/or IL-13Rα2 peptide or composition thereof described herein in accordance with the methods described herein may be an alkylating agent, a nitrosourea, an antimetabolite, and anthracyclin, a topoisomerase II inhibitor, or a mitotic inhibitor. Alkylating agents include, but are not limited to, busulfan, cisplatin, carboplatin, cholormbucil, cyclophosphamide, ifosfamide, decarbazine, mechlorethamine, melphalan, and temozolomide. Nitrosoureas include, but are not limited to carmustine (BCNU) and lomustine (CCNU). Antimetabolites include but are not limited to 5-fluorouracil, capecitabine, methotrexate, gemcitabine, cytarabine, and fludarabine. Anthracyclins include but are not limited to daunorubicin, doxorubicin, epirubicin, idarubicin, and mitoxantrone. Topoisomerase II inhibitors include, but are not limited to, topotecan, irinotecan, etopiside (VP-16), and teniposide. Mitotic inhibitors include, but are not limited to taxanes (paclitaxel, docetaxel), and the vinca alkaloids (vinblastine, vincristine, and vinorelbine).

Currently available cancer therapies and their dosages, routes of administration and recommended usage are known in the art and have been described in such literature as the Physician's Desk Reference (60th ed., 2006).

### 6. EXAMPLES

The following examples further illustrate the invention but, of course, should not be construed as in any way limiting its scope.

### 6.1 EXAMPLE 1

This example demonstrates that EphA2 and IL-13Rα2 are cancer stem cell antigens.

### 6.1.1 Materials and Methods

Flow cytometry was performed on the brain cancer cell line A-172 to assess the expression of EphA2 and IL-13Rα2 on these cancer cells. The experimental protocol included the following steps.

A-172 cells were thawed and plated in 10 cm culture dishes under sterile conditions and using aseptic technique. The A-172 cells were grown in MEM containing 10% FBS. Both cell lines were grown at 37°C with 5% CO₂ in humidified air. The A-172 cells were passaged 1:5 every 3 days.

On the day of the experiments, the cells were washed once with 1x PBS and incubated for 3 minutes with 2 ml 0.25% trypsin-EDTA at 37°C. The cells were then detached from the tissue culture plates with gentle agitation and diluted with 10 ml of DMEM. The cells then were placed in a 50 ml conical tube and centrifuged at 350 × g for 5 minutes. The supernatant was aspirated and the cells were resuspended in 10 ml DMEM. Fifty µl of the cells were mixed with an equal volume of trypan blue and the mixture was carefully placed on a hemacytometer for counting. The cell volumes were then adjusted with DMEM to a concentration of 5×10⁶/ml.

Twenty flow cytometry tubes Fisher Scientific) were prepared and 100 µl of the cells were added to each tube (5 × 10⁵ cells/tube) (10 tubes with A-172 cells).

Twenty µl of Fc blocking reagent was added to each tube and the tubes were incubated at room temperature for 10 minutes.

Ten µl of each antibody, as provided in Table 1, below, was diluted to the described working concentration provided in Table 2, below, and was added to each appropriate tube. The tubes were incubated for 30 minutes at 4°C with gentle agitation.

**TABLE 1: A-172 CELLS**

| **Tube** | **#1** | **#2-3** | **#4-5** | **#6** | **#7** | **#8** | **#9** | **#10** |
|---|---|---|---|---|---|---|---|---|
| **Primary Antibody** | Unstained | Isotype control | Secondary Antibodies Alone | α-CD133 | α-IL13Rα2 | α-EphA2 | α-CD133 + α-IL13Rα2 | α-CD133 + α-EphA2 |
| **Secondary Antibody** | | Anti-mouse OR Anti-goat | Anti-mouse OR Anti-goat | Anti-mouse | Anti-goat | Anti-goat | Anti-mouse + Anti-goat | Anti-mouse + Anti-goat |

**TABLE 2:**

| **Antibody** | **Working Concentration** |
|---|---|
| CD133 | 16.5 µg/ml |
| IL13Rα2 | 10 µg/ml |
| EphA2 | 50 µg/ml |
| Anti-mouse-APC | 1:200 |
| Anti-goat-FITC | 1:200 |

After the incubation, the cells were centrifuged at 300 × g for 1 minute in a tabletop, refrigerated microcentrifuge. The supernatant was removed and the cells were washed with ice cold FACS buffer 3 times. The cells were then resuspended in 100 µl of FACS buffer and 10 µl of the secondary antibodies was added to the appropriate tubes. The tubes were incubated for 30 minutes at 4°C with gentle agitation in the dark.

After the incubation, the cells were centrifuged at 300 × g for 1 minute in a tabletop, refrigerated microcentrifuge. The supernatant was removed and the cells were washed with ice cold FACS buffer 3 times. The cells were then resuspended in 200 µl of FACS buffer and analyzed on a FACSCalibur (BD Biosciences) flow cytometer.

### 6.1.2 Results

In brain cancer, the brain cancer stem cells can be identified using the marker CD133, i.e., brain cancer stem cells are known to express the CD133 antigen (see, e.g., Singh et al., 2004, Nature 432:396-401). The cancer stem cells of the brain cancer cell line A-172 express CD133 (see, e.g., Qiang et al., 2009, Cancer Letters 271:13-21).

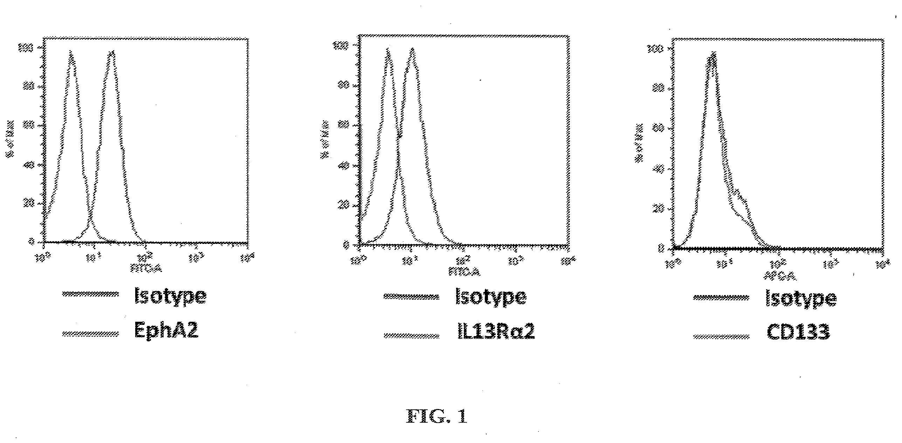

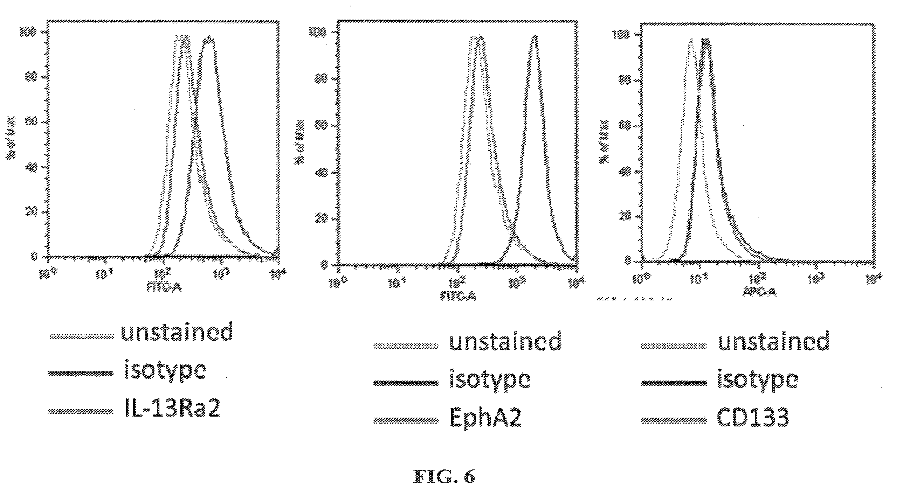

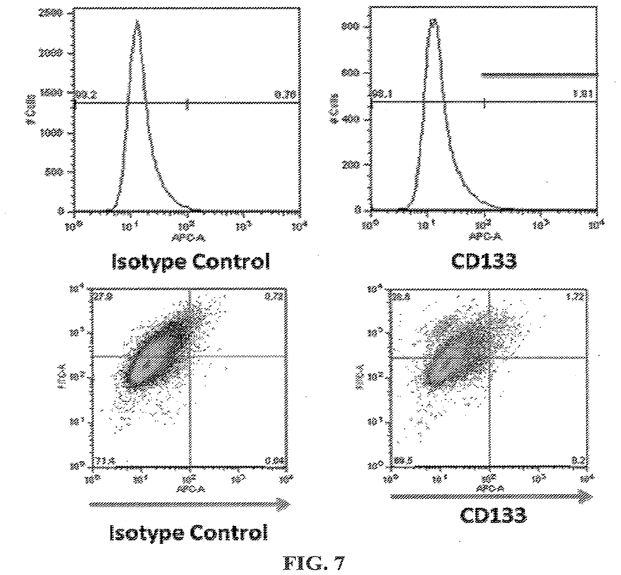

As demonstrated in Figure 1, all cells of the A-172 line were positive for EphA2 and IL-13Rα2, whereas a small population of such cells also were positive for CD133. This CD133+ cell subpopulation thus represents the cancer stem cell subpopulation of the A-172 cell line, and the same expression pattern of CD133 on A-172 cells was observed in a subsequent duplicate experiment (see Figures 6 and 7).

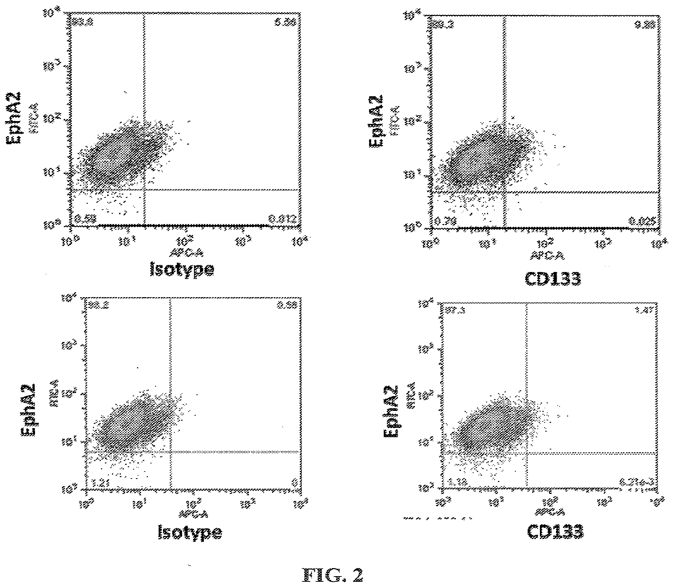

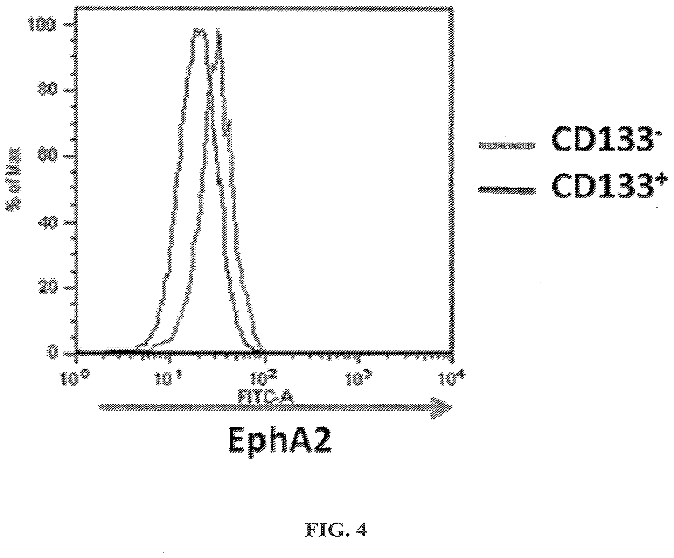

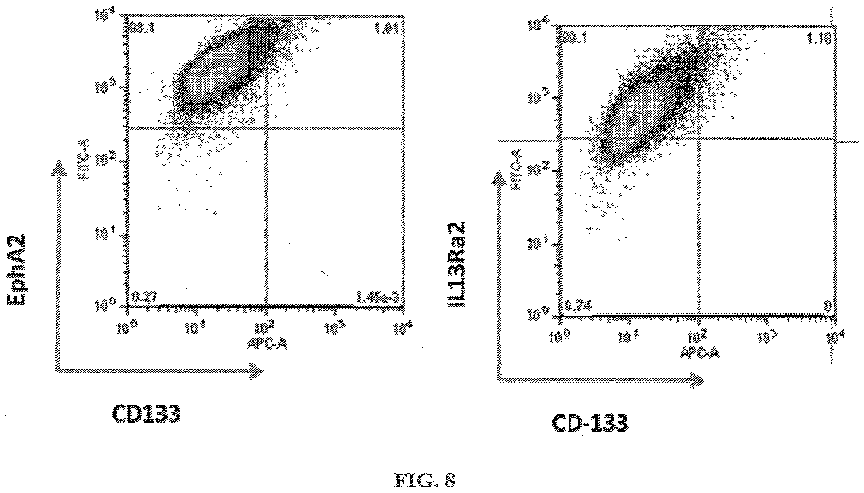

As demonstrated in Figure 2, the CD133+ population also was positive for expression of EphA2, thus demonstrating that EphA2 is present on the cancer stem cell population obtained from the A-172 cell line, and thus that EphA2 is a cancer stem cell antigen. This fact was verified in a subsequent duplicate experiment (see Figure 8). Moreover, as shown in Figure 4, EphA2 was expressed to higher levels on CD133+ cells as compared to CD133- cells

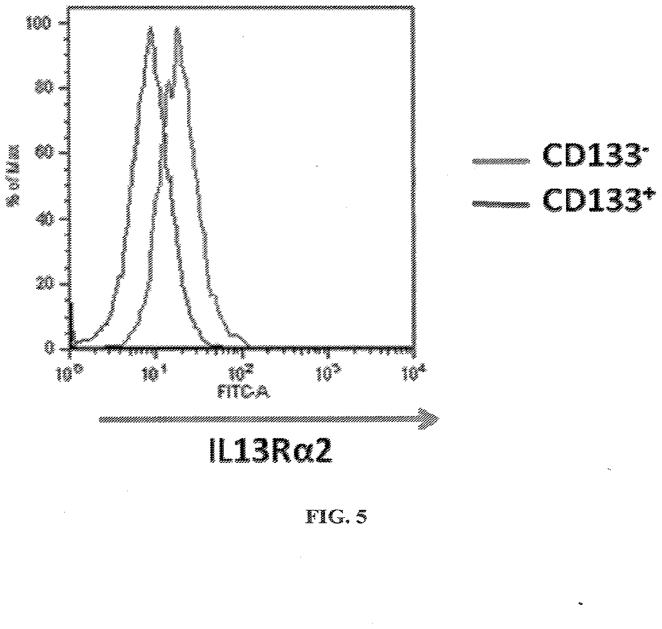

Similarly, as demonstrated by Figure 2, the CD133+ population of A-172 cell line also was positive for expression of IL-13Rα2, thus demonstrating that IL-13Rα2 is present on the cancer stem cell population obtained from the A-172 cell line, and thus that IL-13Rα2 is a cancer stem cell antigen. This fact was verified in a subsequent duplicate experiment (see Figure 8). Moreover, as shown in Figure 5, IL-13Rα2 was expressed to higher levels on CD133+ cells as compared to CD133- cells.

### 6.1.3 Conclusion

These data demonstrate that EphA2 is a cancer stem antigen, and thus can be used in methods for the treatment of cancer, such as brain cancer.

## Claims

1. A composition comprising an IL-13Rα2 peptide and an EphA2 peptide for use in a method of treating, preventing, or managing cancer in a subject in need thereof comprising
(i) administering said composition to said subject and
(ii) measuring the amount of cancer stem cells in said subject.

2. The composition for use according to claim 1, wherein the method further comprises
(iii) comparing the amount of cancer stem cells in a sample obtained from the patient to the amount of cancer stem cells in a reference sample, or to a predetermined reference range, wherein a stabilization or a decrease in the amount of cancer stem cells in the sample relative to the reference sample, or to a predetermined reference range, indicates that the method is effective.

3. The composition for use according to claim 1 or 2, wherein said IL-13Rα2 peptide and said EphA2 peptide are loaded on dendritic cells.

4. The composition for use according to claim 1 or 2, wherein:
(a) said IL-13Rα2:
(i) is a T cell epitope of IL-13Rα2, preferably wherein said T cell epitope of IL-13Rα2 induces an immune response in a subject; or
(ii) comprises any one of SEQ ID NOs: 2, 3, 4, or 5, or consists of any one of SEQ ID NOs: 2, 3, 4, or 5;
and/or
(b) said EphA2:
(i) is a T cell epitope of EphA2, preferably wherein said T cell epitope of EphA2 induces an immune response in a subject; or
(ii) comprises SEQ ID NOs: 1, or consists of SEQ ID NO: 1.

5. An *in vitro* method for monitoring the efficacy of an IL-13Rα2 and EphA2 peptide-based cancer therapy in a patient with cancer, the method comprising:
(a) measuring the amount of cancer stem cells taken from the patient before and following the administration of the IL-13Rα2 and EphA2 peptide-based cancer therapy; and
(b) comparing the amount of cancer stem cells taken from the patient before the administration of the cancer therapy to the amount of cancer stem cells taken from the patient following the administration of the IL-13Rα2 and EphA2 peptide-based cancer therapy, wherein the cancer therapy is determined to be efficacious if the amount cancer stem cells taken from the patient following the administration of the cancer therapy is equivalent to or less than the amount of cancer stem cells taken from the patient before the administration of the cancer therapy.

6. The composition for use according to claims 1 to 4 or the method according to claim 5, wherein said amount of cancer stem cells is measured by using a biopsy, a biological fluid, a bone marrow biopsy, a tumor biopsy, or a normal tissue biopsy from the subject or patient, respectively.

7. The composition for use according to claims 1 to 4 or the method according to claim 5, wherein said amount of cancer stem cells is measured by determining the amount of IL-13Rα2-expressing cancer stem cells and/or EphA2-expressing cancer stem cells, or CD 133-expressing cancer stem cells.

8. The composition for use according to claims 1 to 4 or the method according to claim 5, wherein said amount of cancer stem cells is measured by:
(i) using an immunoassay, wherein the immunoassay is chosen from western blots, immunohistochemistry, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, immunofluorescence, protein A immunoassays, flow cytometry and FACS analysis;
(ii) using a flow cytometer, wherein the amount of cancer stem cells is preferably determined with one or more antibodies that bind cell surface markers, or wherein the cancer stem cells are preferably contacted with one or more dyes prior to detection in the flow cytometer;
(iii) immunohistochemistry;
(iv) using a sphere forming assay;
(v) culturing a sample obtained from the subject, or a portion thereof, and quantitating the cells in an in vitro assay;
(vi) using an immunocompromised mouse in vivo engraftment assay; or
(vii) using imaging, wherein said imaging is preferably MRI, PET, FDG-PET, CT scan, or X-RAY.

9. The composition for use according to claims 1 to 4 or the method according to claim 5, wherein said cancer stem cells are associated with a brain cancer.

10. The composition for use or the method according to claim 9, wherein said brain cancer is glioma, glioblastoma, oligodendroglioma, brain stem glioma, non-brain stem glioma, ependymoma, acoustic schwannoma, cranialpharyngioma, meningioma, medulloblastoma, primary central nervous system lymphoma, tumors of the pineal and pituitary glands, adult low-grade infiltrative supratentorial astrocytoma/oligodendroglioma, adult low-grade infiltrative supratentorial astrocytoma, adult low-grade infiltrative supratentorial oligodendroglioma, adult low-grade infiltrative supratentorial astrocytoma/oligodendroglioma (excluding pilocytic astrocytoma), adult low-grade infiltrative supratentorial astrocytoma (excluding pilocytic astrocytoma), adult low-grade infiltrative supratentorial oligodendroglioma (excluding pilocytic astrocytoma), adult intracranial ependymoma, adult intracranial ependymoma (excluding subependymoma and myxopapillary), adult intracranial anaplastic ependymoma, anaplastic glioma, anaplastic glioblastoma, pilocytic astrocytoma, subependymoma, myxopapillary, leptomeningeal metastases, primary CNS lymphoma, metastatic spine tumor, or meningioma.

11. The composition for use or the method according to claim 10, wherein said glioma is astrocytoma, pilocytic astrocytoma, diffuse astrocytoma, anaplastic astrocytoma, high risk WHO Grade II astrocytoma, oligo astrocytoma, recurrent malignant glioma, recurrent WHO Grade II glioma, newly-diagnosed malignant or intrinsic brain stem glioma, incompletely resected non-brainstem glioma, or recurrent unresectable low-grade glioma.

## Patentansprüche

1. Zusammensetzung, umfassend ein IL-13Rα2-Peptid und ein EphA2-Peptid zur Verwendung in einem Verfahren zum Behandeln, Vorbeugen oder Bewältigen von Krebs bei einem Subjekt, das dies benötigt, umfassend
(i) Verabreichen der Zusammensetzung an das Subjekt und
(ii) Messen der Menge von Krebsstammzellen bei dem Subjekt.

2. Zusammensetzung zur Verwendung nach Anspruch 1, wobei das Verfahren ferner umfasst
(iii) Vergleichen der Menge von Krebsstammzellen in einer Probe, die von dem Patienten erhalten wird, mit der Menge von Krebsstammzellen in einer Referenzprobe oder mit einem vorbestimmten Referenzbereich, wobei eine Stabilisierung oder eine Abnahme in der Menge von Krebsstammzellen in der Probe relativ zu der Referenzprobe oder zu einem vorbestimmten Referenzbereich anzeigt, dass das Verfahren wirksam ist.

3. Zusammensetzung zur Verwendung nach Anspruch 1 oder 2, wobei das IL-13Rα2-Peptid und das EphA2-Peptid auf dendritische Zellen geladen sind.

4. Zusammensetzung zur Verwendung nach Anspruch 1 oder 2, wobei:
(a) das IL-13Rα2:
(i) ein T-Zell-Epitop von IL-13Rα2 ist, vorzugsweise wobei das T-Zell-Epitop von IL-13Rα2 bei einem Subjekt eine Immunantwort induziert; oder
(ii) eine beliebige von SEQ ID NOs: 2, 3, 4 oder 5 umfasst oder aus einer beliebigen von SEQ ID NOs: 2, 3, 4 oder 5 besteht;
und/oder
(b) das EphA2:
(i) ein T-Zell-Epitop von EphA2 ist, vorzugsweise wobei das T-Zell-Epitop von EphA2 bei einem Subjekt eine Immunantwort induziert; oder
(ii) SEQ ID NOs: 1 umfasst oder aus SEQ ID NO: 1 besteht.

5. In-vitro-Verfahren zum Überwachen der Wirksamkeit einer Krebstherapie auf Basis eines IL-13Rα2- und EphA2-Peptids bei einem Patienten mit Krebs, das Verfahren umfassend:
(a) Messen der Menge von Krebsstammzellen, die dem Patienten vor und nach der Verabreichung der Krebstherapie auf Basis des IL-13Rα2- und EphA2-Peptids entnommen wurde; und
(b) Vergleichen der Menge von Krebsstammzellen, die dem Patienten vor der Verabreichung der Krebstherapie entnommen wurde, mit der Menge von Krebsstammzellen, die dem Patienten nach der Verabreichung der Krebstherapie auf Basis des IL-13Rα2- und EphA2-Peptids entnommen wurde, wobei die Krebstherapie als wirksam bestimmt wird, falls die Menge von Krebsstammzellen, die dem Patienten nach der Verabreichung der Krebstherapie entnommen wird, der Menge von Krebsstammzellen entspricht, die dem Patienten vor der Verabreichung der Krebstherapie entnommen wurde, oder geringer als diese ist.

6. Zusammensetzung zur Verwendung nach den Ansprüchen 1 bis 4 oder Verfahren nach Anspruch 5, wobei die Menge von Krebsstammzellen durch Verwenden einer Biopsie, eines biologischen Fluids, einer Knochenmarkbiopsie, einer Tumorbiopsie oder einer normalen Gewebebiopsie von dem Subjekt beziehungsweise Patienten gemessen wird.

7. Zusammensetzung zur Verwendung nach den Ansprüchen 1 bis 4 oder Verfahren nach Anspruch 5, wobei die Menge von Krebsstammzellen durch ein Bestimmen der Menge von IL-13Rα2-exprimierenden Krebsstammzellen und/oder EphA2-exprimierenden Krebsstammzellen oder CD 133-exprimierenden Krebsstammzellen gemessen wird.

8. Zusammensetzung zur Verwendung nach den Ansprüchen 1 bis 4 oder Verfahren nach Anspruch 5, wobei die Menge von Krebsstammzellen gemessen wird durch:
(i) Verwenden eines Immunoassays, wobei der Immunoassay ausgewählt ist aus Western Blots, Immunhistochemie, Radioimmunoassays, ELISA (enzymgekoppelter Immunosorbens-Assay), "Sandwich"-Immunoassays, Immunopräzipitation-Assays, Präzipitinreaktionen, Geldiffusions-Präzipitinreaktionen, Immunodiffusion-Assays, Agglutination-Assays, Komplementfixierung-Assays, immunradiometrische Assays, Fluoreszenzimmunoassays, Immunofluoreszenz, Protein-A-Immunoassays, Durchflusszytometrie und FACS-Analyse;
(ii) Verwenden eines Durchflusszytometers, wobei die Menge von Krebsstammzellen vorzugsweise mit einem oder mehreren Antikörpern bestimmt wird, die Zelloberflächenmarker binden, oder wobei die Krebsstammzellen vor einem Nachweis in dem Durchflusszytometer vorzugsweise mit einem oder mehreren Farbstoffen in Kontakt gebracht werden;
(iii) Immunhistochemie;
(iv) Verwenden eines kugelausbildenden Assays;
(v) Kultivieren einer Probe, die von dem Subjekt erhalten wird oder eines Teils davon und Quantifizieren der Zellen in einem In-vitro-Assay;
(vi) Verwenden eines immunsupprimierte Mäuse-In-vivo-Engraftment-Assays; oder
(vii) Verwenden von Bildgebung, wobei die Bildgebung vorzugsweise MRT, PET, FOG-PET, CT-Scan oder Röntgen ist.

9. Zusammensetzung zur Verwendung nach den Ansprüchen 1 bis 4 oder Verfahren nach Anspruch 5, wobei die Krebsstammzellen mit einem Hirnkrebs assoziiert sind.

10. Zusammensetzung zur Verwendung oder Verfahren nach Anspruch 9, wobei der Hirnkrebs Gliom, Glioblastom, Oligodendrogliom, Hirnstammgliom, Nicht-Hirnstammgliom, Ependymom, Akustikusgeschwulst, Kraniopharyngeom, Meningeom, Medulloblastom, primäres Lymphom des zentralen Nervensystems, Tumore der Zirbel- und Hirnanhangdrüse, niedriggradiges infiltratives supratentorielles Astrozytom/Oligodendrogliom bei Erwachsenen, niedriggradiges infiltratives supratentorielles Astrozytom bei Erwachsenen, niedriggradiges infiltratives supratentorielles Oligodendrogliom bei Erwachsenen, niedriggradiges infiltratives supratentorielles Astrozytom/Oligodendrogliom bei Erwachsenen (außer pilozytisches Astrozytom), niedriggradiges infiltratives supratentorielles Astrozytom bei Erwachsenen (außer pilozytisches Astrozytom), niedriggradiges infiltratives supratentorielles Oligodendrogliom bei Erwachsenen (außer pilozytisches Astrozytom), intrakranielles Ependymom bei Erwachsenen, intrakranielles Ependymom bei Erwachsenen (außer Subependymom und myxopapilläres), intrakranielles anaplastisches Ependymom bei Erwachsenen, anaplastisches Gliom, anaplastisches Glioblastom, pilozytisches Astrozytom, Subependymom, myxopapilläres, leptomeningeale Metastasen, primäres ZNS-Lymphom, metastasierter Wirbelsäulentumor oder Meningeom ist.

11. Zusammensetzung zur Verwendung oder Verfahren nach Anspruch 10, wobei das Gliom Astrozytom, pilozytisches Astrozytom, diffuses Astrozytom, anaplastisches Astrozytom, hochriskantes Astrozytom WHO-Grad II, Oligoastrozytom, rezidivierendes malignes Gliom, rezidivierendes Gliom WHO-Grad II, neu diagnostiziertes malignes oder intrinsisches Hirnstammgliom, unvollständig reseziertes Nicht-Hirnstammgliom oder rezidivierendes nicht resezierbares niedriggradiges Gliom ist.

## Revendications

1. Composition comprenant un peptide IL-13Rα2 et un peptide EphA2 destinée à être utilisée dans un procédé de traitement, de prévention ou de gestion d'un cancer chez un sujet qui en a besoin, comprenant
(i) l'administration de ladite composition audit sujet et
(ii) la mesure de la quantité de cellules souches cancéreuses chez ledit sujet.

2. Composition destinée à être utilisée selon la revendication 1, dans laquelle le procédé comprend en outre
(iii) la comparaison de la quantité de cellules souches cancéreuses dans un échantillon prélevé chez un patient à la quantité de cellules souches cancéreuses dans un échantillon de référence, ou à une plage de référence prédéterminée, dans laquelle une stabilisation ou une diminution de la quantité de cellules souches cancéreuses dans l'échantillon par rapport à l'échantillon de référence, ou à une plage de référence prédéterminée, indique que le procédé est efficace.

3. Composition destinée à être utilisée selon la revendication 1 ou 2, dans laquelle ledit peptide IL-13Rα2 et ledit peptide EphA2 sont chargés sur des cellules dendritiques.

4. Composition destinée à être utilisée selon la revendication 1 ou 2, dans laquelle :
(a) ledit IL-13Rα2 :
(i) est un épitope de lymphocyte T de IL-13Rα2, de préférence dans laquelle ledit épitope de lymphocyte T de IL-13Rα2 induit une réponse immunitaire chez un sujet ; ou
(ii) comprend l'une quelconque des SEQ ID N° : 2, 3, 4 ou 5, ou est constitué de l'un quelconque des SEQ ID NO: 2, 3, 4 ou 5 ;
et/ou
(b) ledit EphA2 :
(i) est un épitope de lymphocyte T de EphA2, de préférence dans laquelle ledit épitope de lymphocyte T de EphA2 induit une réponse immunitaire chez un sujet ; ou
(ii) comprend des SEQ ID NO: 1, ou est constitué de SEQ ID NO: 1.

5. Procédé *in vitro* de surveillance de l'efficacité d'une thérapie anticancéreuse à base de peptides IL-13Rα2 et EphA2 chez un patient atteint d'un cancer, le procédé comprenant :
(a) la mesure de la quantité de cellules souches cancéreuses prélevées chez le patient avant et après l'administration de la thérapie anticancéreuse à base de peptides IL-13Rα2 et EphA2 ; et
(b) la comparaison de la quantité de cellules souches cancéreuses prélevées chez le patient avant l'administration de la thérapie anticancéreuse à la quantité de cellules souches cancéreuses prélevées chez le patient après l'administration de la thérapie anticancéreuse à base de peptides IL-13Rα2 et EphA2, dans lequel la thérapie anticancéreuse est déterminée comme étant efficace si la quantité de cellules souches cancéreuses prélevées chez le patient après l'administration de la thérapie anticancéreuse est équivalente ou inférieure à la quantité de cellules souches cancéreuses prélevées chez le patient avant l'administration de la thérapie anticancéreuse.

6. Composition destinée à être utilisée selon les revendications 1 à 4 ou procédé selon la revendication 5, dans lequel la quantité de cellules souches cancéreuses est mesurée à l'aide d'une biopsie, un fluide biologique, une biopsie de moelle osseuse, une biopsie tumorale ou une biopsie de tissu normal du sujet ou patient, respectivement.

7. Composition destinée à être utilisée selon les revendications 1 à 4 ou procédé selon la revendication 5, dans lequel ladite quantité de cellules souches cancéreuses est mesurée par détermination de la quantité de cellules souches cancéreuses exprimant IL-13Rα2 et/ou de cellules souches cancéreuses exprimant EphA2, ou de cellules souches cancéreuses exprimant CD 133.

8. Composition destinée à être utilisée selon les revendications 1 à 4 ou procédé selon la revendication 5, dans lequel ladite quantité de cellules souches cancéreuses est mesurée :
(i) à l'aide d'un immunoessai, dans laquelle l'immunoessai est choisi parmi transfert de protéines, immunohistochimie, dosages radio-immunologiques, ELISA (essai immuno-enzymatique), dosages « sandwichs », dosages d'immunoprécipitation, réactions à la précipitine, réactions à la précipitine par diffusion de gel, essais d'immunodiffusion, essais d'agglutination, essais de fixation de complément, dosages immunoradiométriques, dosages immunologiques fluorescents, immunofluorescence, dosages immunologiques de protéine A, cytométrie de flux et analyse FACS ;
(ii) à l'aide d'un cytomètre de flux, dans laquelle la quantité de cellules souches cancéreuses est de préférence déterminée avec un ou plusieurs anticorps qui se lient aux marqueurs de surface cellulaire, ou dans laquelle les cellules souches cancéreuses sont de préférence mises en contact avec un ou plusieurs colorants avant la détection dans le cytomètre de flux ;
(iii) par immunohistochimie ;
(iv) à l'aide d'un dosage de formation de sphères ;
(v) en cultivant un échantillon obtenu à partir du sujet, ou une partie de celui-ci, et en quantifiant les cellules dans un dosage in vitro ;
(vi) à l'aide d'un dosage de greffe in vivo chez la souris immunodéprimée ; ou
(vii) à l'aide de l'imagerie, ladite imagerie étant de préférence IRM, PET, FDG-PET, tomodensitométrie ou Rayons X.

9. Composition destinée à être utilisée selon les revendications 1 à 4 ou procédé selon la revendication 5, dans lequel lesdites cellules souches cancéreuses sont associées à un cancer du cerveau.

10. Composition destinée à être utilisée ou procédé selon la revendication 9, dans lequel ledit cancer du cerveau est gliome, glioblastome, oligodendrogliome, gliome du tronc cérébral, gliome du tronc non cérébral, épendymome, schwannome acoustique, craniopharyngiome, méningiome, médulloblastome, lymphome primaire du système nerveux central, tumeur du corps pinéal et de l'hypophyse, astrocytome/oligodendrogliome supratentoriel infiltrant de bas grade chez l'adulte, astrocytome supratentoriel infiltrant de bas grade chez l'adulte, oligodendrogliome supratentoriel infiltrant de bas grade chez l'adulte, astrocytome/oligodendrogliome supratentoriel infiltrant de bas grade chez l'adulte (à l'exclusion de l'astrocytome pilocytique), astrocytome supratentoriel infiltrant de bas grade chez l'adulte (à l'exclusion de l'astrocytome pilocytique), oligodendrogliome supratentoriel infiltrant de bas grade chez l'adulte (à l'exclusion de l'astrocytome pilocytique), épendymome intracrânien chez l'adulte, épendymome intracrânien chez l'adulte (à l'exception du sous-épendymome et du myxopapillaire), épendymome anaplasique intracrânien chez l'adulte, gliome anaplasique, glioblastome anaplasique, astrocytome pilocytique, sous-épendymome, myxopapillaire, métastases leptoméningées, lymphome primaire du SNC, tumeur métastatique de la colonne vertébrale, ou méningiome.

11. Composition destinée à être utilisée ou procédé selon la revendication 10, dans lequel ledit gliome est un astrocytome, astrocytome pilocytique, astrocytome diffus, astrocytome anaplasique, astrocytome de grade II à haut risque selon l'OMS, oligoastrocytome, gliome malin récidivant, gliome récidivant de grade II selon l'OMS, gliome du tronc cérébral intrinsèque ou malin nouvellement diagnostiqué, gliome du tronc non cérébral réséqué de manière incomplète ou gliome récidivant de bas grade non résécable.
